# EUROPÄISCHE PATENTSCHRIFT

(11) **EP 3 632 294 B1**
(45) Veröffentlichungstag und Bekanntmachung des Hinweises auf die Patenterteilung: **04.11.2020**
(21) Anmeldenummer: 19201366.2
(22) Anmeldetag: 03.10.2019
(51) Int. Cl.: A61B 1/00

(54) **SYSTEM UND VERFAHREN ZUM HALTEN EINER BILDWIEDERGABEVORRICHTUNG**
SYSTEM AND METHOD FOR HOLDING AN IMAGE REPRODUCTION DEVICE
SYSTÈME ET PROCÉDÉ DE RETENUE D'UN DISPOSITIF DE REPRODUCTION D'IMAGES

(30) Priorität: 03.10.2018 DE 102018124432
(43) Veröffentlichungstag der Anmeldung: 08.04.2020
(73) Patentinhaber: Karl Storz SE & Co. KG, 78532 Tuttlingen (DE)
(72) Erfinder: Fan, Chunman, 78532 Tuttlingen (DE); Fallert, Johannes, 78532 Tuttlingen (DE); Zhang, Yaokun, 78532 Tuttlingen (DE); Ahrens, Thorsten, 78532 Tuttlingen (DE); Wagner, Sebastian, 78532 Tuttlingen (DE)
(74) Vertreter: Wimmer, Stephan

(56) Entgegenhaltungen:
- WO-A1-95/01749
- WO-A1-2006/046809
- WO-A2-98/46120
- DE-A1-102008 057 734
- KR-A- 20150 135 752
- US-B1- 6 471 637

## Beschreibung

Die vorliegende Erfindung ist auf ein System zum Halten einer Bildwiedergabevorrichtung und auf ein Verfahren zum Wiedergeben eines Bilds bezogen.

Bei mikroinvasiven medizinischen Maßnahmen wird zunehmend der unmittelbare Blick durch ein Endoskop durch eine Kamera und eine Wiedergabe des durch die Kamera erfassten Bilds auf einem oder mehreren Bildschirmen ersetzt. Dies ermöglicht dem medizinischen Personal eine ergonomisch günstigere und weniger ermüdende Körperhaltung. Ferner können mehrere Mitglieder eines medizinischen Teams gleichzeitig die medizinische Maßnahme auf einem oder mehreren Bildschirmen beobachten. Ferner können die Bilder der medizinischen Maßnahme zu Dokumentationszwecken aufgezeichnet und/oder für Ausbildungszwecke beispielsweise in einen Hörsaal übertragen werden.

Die Person, die das Endoskop unmittelbar manuell führt oder eine motorische Bewegung des Endoskops steuert, kennt in der Regel jederzeit Position und Orientierung des Endoskops und seine Blickrichtung. Alle anderen Beteiligten sind auf verbale Hinweise oder auf die eigene Beobachtung der Bewegungen des wiedergegebenen Bilds sowie die eigenen anatomischen Kenntnisse angewiesen.

Um medizinisches Personal bei dem Zurechtfinden im wiedergegebenen Bild, insbesondere beim möglichst intuitiven Erfassen der momentanen Orientierung des Endoskops und der Blickrichtung zu unterstützen, wurde bereits vorgeschlagen, Hilfsobjekte in das Bild einzublenden, die beispielsweise eine vorbestimmte und feststehende Referenzrichtung andeuten. Ferner wurde bereits vorgeschlagen, das auf dem Bildschirm wiedergegebene Bild entsprechend der Orientierung des Endoskops zu rotieren oder zu verformen.

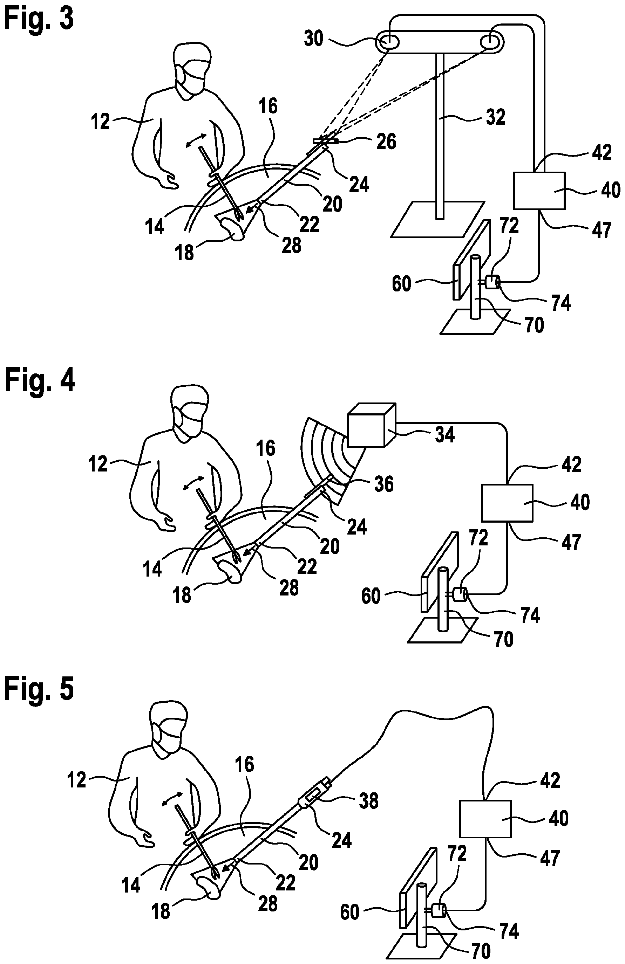

In WO 95/01749 A1 (auch veröffentlicht als EP 0 712 289 B1) ist eine Vorrichtung zum Drehen einer Bildwiedergabeeinrichtung beschrieben, die eine Orientierung des wiedergegebenen Bilds, die der tatsächlichen Orientierung entspricht, ermöglicht (Seite 2, Zeilen 20 bis 26). Die Ablenkelektroden einer Kathodenstrahlröhre eines Bildschirms sind an einem Rahmen angeordnet, der mittels eines Servomotor 22 rotiert werden kann (Seite 5, Zeilen 26 bis 33, Figur 5).

In US 8,187,167 B2 (auch veröffentlicht als WO 2006/046809 A1; ähnlich: US 2012-0296164 A1) ist eine Anzeigevorrichtung für die Laparoskopie beschrieben. Die Anzeigevorrichtung umfasst einen Bildschirm zum Wiedergeben eines mittels eines Laparoskops erfassten Bilds. Der Bildschirm ist motorisch um die Flächennormale seiner Bildwiedergabefläche rotierbar. Die Anzeigevorrichtung umfasst ferner einen Fußschalter, mittels dessen medizinisches Personal eine Rotation des Bildschirms steuern kann.

Eine Aufgabe der vorliegenden Erfindung besteht darin, ein verbessertes System zum Halten einer Bildwiedergabevorrichtung und ein verbessertes Verfahren zum Wiedergeben eines mittels einer bewegbaren Bilderfassungsvorrichtung erfassten Bilds zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, einen Bildschirm, einen Monitor oder eine andere Bildwiedergabevorrichtung, die ein mittels eines Endoskops, eines Exoskops oder einer anderen Bilderfassungsvorrichtung erfasstes Bild wiedergibt, so zu bewegen, dass die Orientierung der Bildwiedergabevorrichtung im Raum jederzeit der Orientierung der Bilderfassungsvorrichtung im Raum entspricht oder weitgehend entspricht oder diese zumindest andeutet.

Ein System zum Halten einer Bildwiedergabevorrichtung zum Wiedergeben eines mittels einer Bilderfassungsvorrichtung erfassten Bilds umfasst eine bewegbare Haltevorrichtung zum veränderbaren Halten der Bildwiedergabevorrichtung, eine steuerbare Antriebseinrichtung zum Bewegen der Haltevorrichtung mit einem Steuersignaleingang zum Empfangen eines Steuersignals und eine Steuerung mit einem Signaleingang zum Empfangen eines Signals, das eine Orientierung oder eine Änderung der Orientierung der Blickrichtung der Bilderfassungsvorrichtung im Raum repräsentiert oder eine Bestimmung der Orientierung oder der Änderung der Orientierung der Blickrichtung der Bilderfassungsvorrichtung ermöglicht, und einem mit dem Steuersignaleingang der steuerbaren Antriebseinrichtung koppelbaren Steuersignalausgang zum Bereitstellen eines Steuersignals zum Steuern der steuerbaren Antriebseinrichtung, wobei die Steuerung vorgesehen und ausgebildet ist, um die steuerbare Antriebseinrichtung so zu steuern, dass innerhalb eines vorbestimmten Bereichs möglicher Orientierungen der Blickrichtung der Bilderfassungsvorrichtung im Raum die Orientierung der Bildwiedergabevorrichtung im Raum eine vorbestimmte Funktion der Orientierung der Blickrichtung der Bilderfassungsvorrichtung im Raum ist.

Die Bilderfassungsvorrichtung und die Bildwiedergabevorrichtung sind insbesondere jeweils nicht Bestandteil des Systems. Alternativ kann das System jedoch eine Bilderfassungsvorrichtung umfassen und für die Kombination mit einer Bildwiedergabevorrichtung, die nicht Bestandteil des Systems ist, vorgesehen und ausgebildet sein; oder eine Bildwiedergabevorrichtung umfassen und für die Kombination mit einer Bilderfassungsvorrichtung, die nicht Bestandteil des Systems ist, vorgesehen und ausgebildet sein; oder sowohl eine Bilderfassungsvorrichtung als auch eine Bildwiedergabevorrichtung umfassen.

Die Bilderfassungsvorrichtung ist insbesondere ein Endoskop, ein Exoskop, ein Operationsmikroskop oder eine andere Vorrichtung zum Erfassen eines monokularen oder eines stereoskopischen Bilds. Die Bilderfassungsvorrichtung kann eine oder mehrere Kameras oder einen oder mehrere Bildsensoren zum Erfassen eines Bilds oder eines Stereobilds und zum Erzeugen eines das Bild oder Stereobild repräsentierenden Bildsignals umfassen. Alternativ kann die Bilderfassungsvorrichtung vorgesehen und ausgebildet sein, um mit einer oder mehreren Kameras oder einem oder mehreren Bildsensoren optisch gekoppelt zu werden, beispielsweise über ein Okular.

Die Bildwiedergabevorrichtung umfasst insbesondere einen (oder mehrere) Bildschirm oder Monitor oder einen (oder mehrere) Projektor mit einer (oder mehreren) zugeordneten Projektionsfläche.

Die Haltevorrichtung kann zur dauerhaften, d. h. nicht zerstörungsfrei oder nur mittels Werkzeugs lösbaren Verbindung mit einer Bildwiedergabevorrichtung vorgesehen und ausgebildet sein. Alternativ kann die Haltevorrichtung zur leicht lösbaren und insbesondere auch durch medizinisches Personal und/oder ohne Verwendung von Werkzeug lösbaren Verbindung mit einer Bildwiedergabevorrichtung vorgesehen und ausgebildet sein.

Die Haltevorrichtung kann einen oder mehrere rotatorische Freiheitsgrade und optional einen oder mehrere translatorische Freiheitsgrade aufweisen. Die Haltevorrichtung umfasst insbesondere für jeden rotatorischen Freiheitsgrad ein oder mehrere Gelenke und für jeden translatorischen Freiheitsgrad eine oder mehrere Linearführungen oder Linearlager, die beispielsweise eine Teleskopierbarkeit ermöglichen. Alternativ kann ein translatorischer Freiheitsgrad mittels eines oder mehrerer Gelenke realisiert sein. Diese Gelenke können wiederum gleichzeitig rotatorische Freiheitsgrade ermöglichen.

Die bewegbare Haltevorrichtung kann jeweils ein Gelenk, das eine horizontale Schwenk- oder Rotationsachse definiert, und ein Gelenk, das eine vertikale Schwenk- oder Rotationsachse definiert, aufweisen. Insbesondere umfasst die bewegbare Haltevorrichtung ein Gelenk, das eine jederzeit vertikale Schwenk- oder Rotationsachse definiert, ein Gelenk, das eine jederzeit horizontale Schwenk- oder Rotationsachse, die um die vertikale Schwenkachse schwenkbar ist, definiert, und ein Gelenk, das eine dritte Schwenkachse, die jederzeit zu der vertikalen ersten Schwenkachse und zu der jederzeit horizontalen zweiten Schwenkachse orthogonal ist, definiert.

Ferner kann die bewegbare Haltevorrichtung mehrere Linearführungen oder Linearlager aufweisen, die jeweils einen geraden oder gekrümmten Pfad definieren. In diesem Fall kann eine erste Linearführung ortsfest angeordnet sein, wobei eine oder mehrere weitere Linearführungen entlang des durch die erste Linearführung definierten Pfads bewegbar sind. Beispielsweise kann eine erste Linearführung der Haltevorrichtung einen geraden horizontalen Translationspfad definieren und eine zweite Linearführung einen geraden vertikalen Translationspfad definieren.

Alternativ kann die bewegbare Haltevorrichtung so ausgebildet sein, dass nicht oder nur in einzelnen Konfigurationen der Haltevorrichtung alle Schwenk- oder Rotationsachsen und Translationspfade orthogonal zueinander und entweder vertikal (d. h. in Richtung der Gravitation) oder horizontal (d. h. orthogonal zur Vertikalen) orientiert sind. Insbesondere kann die Haltevorrichtung einen oder mehrere Hexapoden umfassen und/oder ähnlich den Vorrichtungen, die gewöhnlich als Industrieroboter bezeichnet werden, ausgebildet sein. Ein Hexapod und eine Ausgestaltung in der Art eines Industrieroboters mit mehreren Gelenken, die jeweils eine Schwenk- oder Rotationsachse definieren, sind Beispiele, bei denen auch translatorische Freiheitsgrade allein durch Gelenke verwirklicht werden.

Die steuerbare Antriebseinrichtung kann in die Haltevorrichtung teilweise oder vollständig integriert sein. Die steuerbare Antriebseinrichtung kann einen oder mehrere elektrische, pneumatische, hydraulische oder andere Motoren oder Aktoren sowie ein oder mehrere Getriebe umfassen. Insbesondere ist jedem Freiheitsgrad der bewegbaren Haltevorrichtung ein Motor oder Aktor zugeordnet.

Die Steuerung kann zur analogen oder digitalen, elektrischen, optischen oder anderen Verarbeitung von ursprünglich elektrischen, optischen oder anderen analogen oder digitalen Signalen und zur Erzeugung von elektrischen, optischen oder anderen analogen oder digitalen Signalen vorgesehen und ausgebildet sein. In die Steuerung kann eine Leistungsversorgung zur Bereitstellung elektrischer, pneumatischer, hydraulischer oder anderer Leistung für einen oder mehrere Motoren oder Aktoren der Antriebseinrichtung integriert sein. Alternativ kann die Steuerung lediglich zur Ansteuerung einer oder mehrerer Leistungsversorgungen für elektrische Motoren oder Aktoren oder zur Ansteuerung von Ventilen vorgesehen und ausgebildet sein. Die Steuerung kann mit der Antriebseinrichtung und/oder mit der Haltevorrichtung integriert sein.

Mit der Orientierung der Blickrichtung der Bilderfassungsvorrichtung oder der Bildwiedergabevorrichtung im Raum ist insbesondere die Orientierung der Vorrichtung bezogen auf ein beliebiges vorbestimmtes Koordinatensystem gemeint. Als Koordinatensystem kommt beispielsweise ein kartesisches Koordinatensystem mit zwei horizontalen und einer vertikalen Achse in Betracht. Vertikal ist die Richtung der am Ort des Systems wirkenden Gravitationskraft, horizontal ist jede dazu orthogonale Richtung. Die horizontalen Achsen des Koordinatensystems sind beispielsweise Tangenten an die Breiten- und Längenkreise des globalen sphärischen Koordinatensystems der Erdoberfläche. Alternativ können eine oder mehrere Achsen des Koordinatensystems parallel zu Kanten eines quaderförmigen Raums, in dem das System angeordnet ist, oder zu den Grundrichtungen (beispielsweise dorsal - kaudal und lateral) eines Patienten verwendet werden.

Die Orientierung einer Vorrichtung im Raum kann beispielsweise durch Winkel zwischen Bezugsrichtungen der Vorrichtung und Achsen des Koordinatensystems oder zwischen Bezugsrichtungen der Vorrichtung und durch die Achsen eines Koordinatensystems aufgespannte Ebenen beschrieben werden.

Eine Bezugsrichtung der Bilderfassungsvorrichtung ist insbesondere ihre Blickrichtung. Als weitere Bezugsrichtung einer Bilderfassungsvorrichtung kann die Richtung eines geraden Randabschnitts eines rechteckigen Bilds oder Bildfelds der Bilderfassungsvorrichtung verwendet werden. Im Falle einer Bilderfassungsvorrichtung, die, wie beispielsweise viele Endoskope, ein kreisförmiges Bild erzeugt, kann ein gerader Randabschnitt eines Bereichs, der durch eine Kamera, die mit der Bilderfassungsvorrichtung gekoppelt ist, erfasst wird, verwendet werden.

Die Blickrichtung der Bilderfassungsvorrichtung ist die Richtung von einer Lichteintrittsfläche (insbesondere am distalen Ende) der Bilderfassungsvorrichtung zu einem weit entfernten Objekt, das in der Mitte des Sichtfelds liegt und somit in die Mitte des Bildfelds abgebildet wird. Im Fall eines Bildsensors, dessen Bildsignale nicht vollständig verwendet werden, ist die Blickrichtung diejenige Richtung, in der ein weit entferntes Objekt liegt, das in die Mitte desjenigen Bereichs des Bildsensors, dessen Bildsignale tatsächlich weitergeleitet und an der Bildwiedergabevorrichtung wiedergegeben werden, abgebildet wird. Die Blickrichtung einer Bilderfassungsvorrichtung kann in Bezug auf die Bilderfassungsvorrichtung veränderbar, insbesondere schwenkbar sein, beispielsweise im Fall eines Endoskops mit einem Schwenkprisma oder mit einer schwenkbaren Kamera an seinem distalen Ende.

Als Bezugsrichtungen einer Bildwiedergabevorrichtung können beispielsweise die Flächennormale oder - im Fall einer gekrümmten Bildwiedergabefläche - die mittlere Flächennormale der Bildwiedergabefläche oder die Flächennormale der Bildwiedergabefläche der Bildwiedergabevorrichtung in deren Mitte und ein gerader Abschnitt eines rechteckigen Rands der Bildwiedergabefläche verwendet werden.

Die Orientierung einer Vorrichtung kann insbesondere durch einen Azimutalwinkel (auch als Horizontalwinkel bezeichnet), einen Zenitwinkel (auch als Elevation oder, abhängig vom Vorzeichen, als Höhenwinkel oder Tiefenwinkel bezeichnet) und einen dritten Winkel (im Folgenden als Neigungswinkel bezeichnet) angegeben werden. Der Azimutalwinkel wird innerhalb einer horizontalen Ebene in einer bestimmten Richtung (insbesondere von oben gesehen im Uhrzeigersinn) von einer bestimmten Richtung (beispielsweise Norden) gemessen. Der Zenitwinkel wird als Winkel zur Vertikalen gemessen. Die Elevation oder der Höhenwinkel wird als Winkel zur horizontalen Ebene gemessen, bei negativem Vorzeichen wird auch der Begriff Tiefenwinkel verwendet. Die Orientierung einer Vorrichtung kann beispielsweise als Azimutalwinkel, Zenitwinkel und Neigungswinkel der Vorrichtung gegenüber einer Bezugsorientierung der Vorrichtung angegeben werden.

Der vorbestimmte Bereich möglicher Orientierungen der Bilderfassungsvorrichtung im Raum, innerhalb dessen die Orientierung der Bildwiedergabevorrichtung eine vorbestimmte Funktion der Orientierung der Bilderfassungsvorrichtung ist, kann eine Mannigfaltigkeit mit der Dimension 1, 2 oder 3 sein. Beispielsweise kann die Bildwiedergabevorrichtung lediglich um eine vertikale Schwenk- oder Rotationsachse schwenkbar sein, wobei die Steuerung vorgesehen und ausgebildet ist, um die Richtung der mittleren Flächennormale der Bildwiedergabefläche der Bildwiedergabevorrichtung innerhalb eines bestimmten Winkelbereichs jederzeit parallel zur Projektion der Blickrichtung der Bilderfassungsvorrichtung auf eine horizontale Ebene zu halten. Alternativ oder zusätzlich kann die Bildwiedergabevorrichtung um eine horizontale Achse (insbesondere parallel zur Bildwiedergabefläche der Bildwiedergabevorrichtung oder orthogonal zur mittleren Flächennormale der Bildwiedergabefläche) schwenkbar sein, wobei die Steuerung vorgesehen und ausgebildet ist, um die Bildwiedergabevorrichtung innerhalb eines vorbestimmten Winkelbereichs jederzeit so zu bewegen, dass der Zenitwinkel für die Blickrichtung der Bilderfassungsvorrichtung und der Zenitwinkel für die mittlere Flächennormale der Bildwiedergabefläche der Bildwiedergabevorrichtung gleich sind.

Grenzen des vorbestimmten Bereichs möglicher Orientierungen der Bilderfassungsvorrichtung, innerhalb dessen die Orientierung der Bildwiedergabevorrichtung eine vorbestimmte Funktion der Orientierung der Blickrichtung der Bilderfassungsvorrichtung ist, ergeben sich beispielsweise aus Grenzen der Bewegbarkeit der Bildwiedergabevorrichtung. Beispielsweise ermöglicht ein Hexapod aus geometrischen Gründen ein Schwenken nur innerhalb eines eingeschränkten Raumwinkelbereichs (dessen Inhalt in der Regel kleiner oder nicht viel größer als 2π (2 Pi) aber in den meisten Fällen deutlich kleiner als 4π (4 Pi) ist) geschwenkt werden. Ferner können Grenzen des vorbestimmten Bereichs resultieren aus der Länge oder Biegeelastizität von Kabeln an der Bildwiedergabevorrichtung und vor allem aus der Bedingung, dass medizinisches Personal ohne einen Standortwechsel das von der Bildwiedergabevorrichtung wiedergegebene Bild uneingeschränkt betrachten können sollen.

Das System ermöglicht eine intuitive Erfassung der momentanen Orientierung der Blickrichtung der Bilderfassungsvorrichtung durch Benutzer oder Betrachter allein durch Wahrnehmung der Orientierung der Bildwiedergabevorrichtung.

Bei einem System, wie es hier beschrieben ist, ist die Steuerung insbesondere ausgebildet, um die Antriebseinrichtung so zu steuern, dass innerhalb eines vorbestimmten Winkelbereichs ein Schwenken der Blickrichtung der Bilderfassungsvorrichtung um eine erste Achse ein Schwenken der Bildwiedergabevorrichtung um eine zweite Achse bewirkt.

Die Steuerung ist insbesondere ausgebildet, um die Antriebseinrichtung so zu steuern, dass innerhalb eines vorbestimmten Winkelbereichs jedes Schwenken der Blickrichtung der Bilderfassungsvorrichtung um eine erste Achse ein Schwenken der Bildwiedergabevorrichtung um eine zweite Achse bewirkt.

Die erste Achse ist insbesondere orthogonal oder im Wesentlichen orthogonal zu der Blickrichtung der Bilderfassungsvorrichtung. Die zweite Achse ist insbesondere orthogonal oder im Wesentlichen orthogonal zu einer Flächennormale oder - im Fall einer gekrümmten Bildwiedergabefläche der Bildwiedergabevorrichtung - zu einer mittleren Flächennormale der Bildwiedergabefläche der Bildwiedergabevorrichtung.

Die erste Achse und die zweite Achse können parallel oder im Wesentlichen parallel sein. Die erste Achse ist insbesondere horizontal und orthogonal zu der Blickrichtung der Bilderfassungsvorrichtung. Alternativ ist die erste Achse beispielsweise orthogonal zu der Blickrichtung der Bilderfassungsvorrichtung und orthogonal zu einer Horizontalen, die ihrerseits orthogonal zu der Blickrichtung ist. Insbesondere kann die erste Achse vertikal sein.

Bei einem System, wie es hier beschrieben ist, ist die Steuerung insbesondere ausgebildet, um die Antriebseinrichtung so zu steuern, dass hinsichtlich eines Schwenkens der Blickrichtung der Bilderfassungsvorrichtung um die erste Achse und eines Schwenkens der Bildwiedergabevorrichtung um die zweite Achse innerhalb des vorbestimmten Winkelbereichs jeder Winkelposition der Blickrichtung der Bilderfassungsvorrichtung eine Winkelposition der Bildwiedergabevorrichtung zugeordnet ist.

Die Zuordnung kann durch eine mathematische Funktion oder durch eine Tabelle (im Englischen oft bezeichnet als look-up-table) definiert sein. Die Zuordnung zwischen der Winkelposition der Blickrichtung der Bilderfassungsvorrichtung und der Winkelposition der Bildwiedergabevorrichtung entspricht insbesondere einer monotonen oder streng monotonen mathematischen Funktion.

Der vorbestimmte Winkelbereich und seine Grenzen können Randbedingungen wie die Länge oder Elastizität eines Kabels oder den zulässigen Schwenkbereich eines Gelenks berücksichtigen. Grenzen des vorbestimmten Winkelbereichs können ferner resultieren aus der Bedingung, dass medizinisches Personal ohne einen Standortwechsel das von der Bildwiedergabevorrichtung wiedergegebene Bild uneingeschränkt betrachten können sollen.

Die Zuordnung ist insbesondere so, dass in der Mitte oder in einem mittleren Bereich des vorbestimmten Winkelbereichs jedes Schwenken der Bilderfassungsvorrichtung um einen kleinen Winkel ein Schwenken der Bildwiedergabevorrichtung um den gleichen Winkel bewirkt. Die Zuordnung ist insbesondere so, dass das Verhältnis zwischen dem Winkel, um den die Bildwiedergabevorrichtung geschwenkt wird, zu dem Winkel, um den die Bilderfassungsvorrichtung geschwenkt wird, zu den Rändern des vorbestimmten Winkelbereichs hin immer kleiner wird. So kann einerseits in der Mitte des vorbestimmten Winkelbereichs die Orientierung der Bildwiedergabevorrichtung im Raum die Orientierung der Bilderfassungsvorrichtung im Raum präzise wiedergeben und andererseits auch an den Rändern des vorbestimmten Winkelbereichs noch eine Betrachtung eines von der Bildwiedergabevorrichtung wiedergegebenen Bilds ermöglicht werden.

Bei einem System, wie es hier beschrieben ist, ist die Steuerung insbesondere ferner ausgebildet, um die Antriebseinrichtung so zu steuern, dass ein Schwenken der Blickrichtung der Bilderfassungsvorrichtung um die erste Achse ferner ein Bewegen der Bildwiedergabevorrichtung entlang eines Pfads bewirkt.

Der Pfad ist insbesondere gekrümmt, beispielsweise kreisbogenförmig. Der Pfad liegt insbesondere in einer Ebene oder im Wesentlichen in einer Ebene orthogonal zu der zweiten Achse und/ oder zu der ersten Achse. Die Steuerung kann ausgebildet sein, um die Antriebseinrichtung so zu steuern, dass die Bildwiedergabevorrichtung gleichzeitig um die zweite Achse geschwenkt und entlang des Pfads bewegt wird. Die Position des Pfads kann ihrerseits abhängig sein von einer Winkelposition der Blickrichtung der Bilderfassungsvorrichtung bezogen auf eine weitere Achse, die insbesondere orthogonal zu der ersten Achse ist. Beispielsweise kann die Bildwiedergabevorrichtung gleichzeitig um eine horizontale Achse und um eine vertikale Achse schwenkbar und auf einer Kugeloberfläche oder einer Oberfläche eines Ellipsoids oder auf einer anderen gekrümmten Fläche bewegbar sein. Dabei ist die Steuerung insbesondere ausgebildet, um die Antriebseinrichtung so zu steuern, dass die mittlere Flächennormale der Bildwiedergabefläche der Bildwiedergabevorrichtung jederzeit zum Krümmungsmittelpunkt der Fläche, entlang der die Bildwiedergabevorrichtung bewegbar ist, in dem Punkt, an dem die Bildwiedergabevorrichtung sich gerade befindet, zeigt. Anders ausgedrückt ist die Steuerung insbesondere ausgebildet, um die Antriebseinrichtung so zu steuern, dass die Bildwiedergabefläche der Bildwiedergabevorrichtung jederzeit parallel ist zur lokalen Tangentialfläche der Fläche, entlang derer die Bildwiedergabevorrichtung bewegbar ist.

Bei einem System, wie es hier beschrieben ist, ist die Steuerung insbesondere ferner ausgebildet, um die Antriebseinrichtung so zu steuern, dass innerhalb des vorbestimmten Winkelbereichs jeder Winkelposition der Blickrichtung der Bilderfassungsvorrichtung eine Position der Bildwiedergabevorrichtung entlang des vorbestimmten Pfads zugeordnet ist.

Bei einem System, wie es hier beschrieben ist, weist die Haltevorrichtung insbesondere ein oder mehrere Gelenke auf, die ein Schwenken der Bildwiedergabevorrichtung um die zweite Achse erlauben, wobei die Antriebseinrichtung einen oder mehrere Motoren oder Aktoren zum Schwenken einer mittels der Haltevorrichtung gehaltenen Bildwiedergabevorrichtung um die zweite Achse umfasst.

Bei einem System, wie es hier beschrieben ist, ist die Steuerung insbesondere ferner ausgebildet, um die Antriebseinrichtung so zu steuern, dass innerhalb eines vorbestimmten Winkelbereichs ein Verändern eines Winkels zwischen der Blickrichtung der Bilderfassungsvorrichtung und der Vertikalen eine Veränderung des Winkels zwischen der Flächennormalen einer Bildwiedergabefläche der Bildwiedergabevorrichtung und der Vertikalen bewirkt.

Die Steuerung ist insbesondere ausgebildet, um die Antriebseinrichtung so zu steuern, dass innerhalb eines vorbestimmten Winkelbereichs jedes Verändern eines Winkels zwischen der Blickrichtung der Bilderfassungsvorrichtung und der Vertikalen eine Veränderung des Winkels zwischen der Flächennormalen einer Bildwiedergabefläche der Bildwiedergabevorrichtung und der Vertikalen bewirkt.

Bei einem System, wie es hier beschrieben ist, ist die Steuerung insbesondere ausgebildet, um die Antriebseinrichtung so zu steuern, dass innerhalb eines vorbestimmten Winkelbereichs ein, insbesondere jedes, Schwenken der Blickrichtung der Bilderfassungsvorrichtung um eine erste horizontale Achse ein Schwenken der Bildwiedergabevorrichtung um eine zweite horizontale Achse bewirkt.

Die zweite horizontale Achse ist insbesondere parallel zu der ersten horizontalen Achse. Die erste horizontale Achse ist insbesondere orthogonal zu der Blickrichtung der Bilderfassungsvorrichtung.

Bei einem System, wie es hier beschrieben ist, ist die Steuerung insbesondere ausgebildet, um die Antriebseinrichtung so zu steuern, dass innerhalb eines vorbestimmten Winkelbereichs jedem Winkel zwischen der Blickrichtung der Bilderfassungsvorrichtung und der Vertikalen ein Winkel zwischen der Flächennormalen der Bildwiedergabefläche der Bildwiedergabevorrichtung und der Vertikalen zugeordnet ist.

Bei einem System, wie es hier beschrieben ist, ist die Steuerung insbesondere ferner ausgebildet, um die Antriebseinrichtung so zu steuern, dass ein Schwenken der Blickrichtung der Bilderfassungsvorrichtung um die erste horizontale Achse ferner ein Bewegen der Bildwiedergabevorrichtung entlang eines Pfads, der zumindest in einem kleinen Abschnitt oder in einem Punkt vertikal oder im Wesentlichen vertikal ist, bewirkt.

Der Pfad ist insbesondere kreisbogenförmig oder anders gekrümmt. Der Pfad liegt insbesondere in einer vertikalen Ebene. Der Pfad liegt insbesondere in einer vertikalen Ebene, die orthogonal zu der ersten horizontalen Achse ist. Die erste horizontale Achse ist insbesondere orthogonal zu der Blickrichtung der Bilderfassungsvorrichtung.

Bei einem System, wie es hier beschrieben ist, ist die Steuerung insbesondere ausgebildet, um die Antriebseinrichtung so zu steuern, dass innerhalb des vorbestimmten Winkelbereichs jeder Winkelposition der Blickrichtung der Bilderfassungsvorrichtung eine Position der Bildwiedergabevorrichtung entlang des vorbestimmten Pfads zugeordnet ist.

Bei einem System, wie es hier beschrieben ist, ist die Steuerung insbesondere ferner ausgebildet, um die Antriebseinrichtung so zu steuern, dass ein translatorisches Bewegen der Bilderfassungsvorrichtung ein translatorisches Bewegen der Bildwiedergabevorrichtung bewirkt.

Bei einem System, wie es hier beschrieben ist, ist der Signaleingang der Steuerung insbesondere ferner zum Empfangen eines Signals, das eine Position oder eine Änderung der Position der Bilderfassungsvorrichtung repräsentiert oder eine Bestimmung der Position oder der Änderung der Position der Bilderfassungsvorrichtung ermöglicht, ausgebildet, wobei die Steuerung ferner vorgesehen und ausgebildet ist, um die steuerbare Antriebseinrichtung so zu steuern, dass innerhalb eines vorbestimmen Bereichs möglicher Positionen der Bilderfassungsvorrichtung die Position der Bildwiedergabevorrichtung eine vorbestimmte Funktion der Position der Bilderfassungsvorrichtung ist.

Mit der Position einer Vorrichtung ist die Position eines Bezugspunkts der Vorrichtung, beispielsweise des Mittelpunkts der Lichteintrittsfläche der Bilderfassungsvorrichtung oder des Mittelpunkts der Bildwiedergabefläche der Bildwiedergabevorrichtung, gemeint. Jede Rotation einer Vorrichtung um ihren Bezugspunkt ändert ihre Position nicht. Eine rein translatorische Bewegung der Vorrichtung ändert ihre Position, jedoch nicht ihre Orientierung.

Der vorbestimmte Bereich kann eine Mannigfaltigkeit mit einer, zwei oder drei Dimensionen sein, insbesondere entsprechend einer gleichen Anzahl von Freiheitsgraden. Die Position der Bildwiedergabevorrichtung kann die Position der Bilderfassungsvorrichtung andeuten, so dass medizinisches Personal aus der Position der Bildwiedergabevorrichtung auf die Position der Bilderfassungsvorrichtung schließen kann.

Bei einem System, wie es hier beschrieben ist, ist der Signaleingang der Steuerung insbesondere zum Empfangen eines Signals oder eines über die Zeit integrierten Signals von einem Sensor ausgebildet.

Der Signaleingang der Steuerung ist insbesondere zum Empfangen eines Signals von einem Sensor zur Erfassung der Richtung der Schwerkraft und/oder zur Erfassung einer linearen Beschleunigung und/oder zur Erfassung einer Rotation und/oder zur Erfassung einer Richtung und/oder einer Stärke eines Magnetfelds vorgesehen und ausgebildet.

Ein System, wie es hier beschrieben ist, umfasst insbesondere ferner einen Sensor zur Erfassung zumindest entweder der Richtung der Schwerkraft oder einer linearen Beschleunigung oder einer Rotation oder einer Richtung oder Stärke eines Magnetfelds.

Ein Sensor zur Erfassung einer Rotation umfasst insbesondere ein Gyroskop, einen Vibrationskreisel oder einen anderen Drehratensensor. Der Sensor ist insbesondere Bestandteil der Bilderfassungsvorrichtung oder mit dieser mechanisch starr verbunden oder verbindbar.

Durch Erfassung der Richtung der Schwerkraft durch einen Sensor in oder an einer Bilderfassungsvorrichtung kann die Orientierung der Bilderfassungsvorrichtung relativ zur Vertikalen bestimmt werden. Durch Erfassung der Richtung des Erdmagnetfelds oder beispielsweise eines in einem Operationsraum künstlich erzeugten Magnetfelds durch einen Sensor, der in oder an einer Bilderfassungsvorrichtung angeordnet ist, kann die Orientierung der Bilderfassungsvorrichtung relativ zu dem Magnetfeld bestimmt werden. Durch Erfassung der Richtung eines künstlich erzeugten elektrischen Felds oder durch Erfassung der Polarisation eines elektromagnetischen Wechselfelds durch einen Sensor, der in oder an einer Bilderfassungsvorrichtung angeordnet ist, kann die Orientierung der Bilderfassungsvorrichtung relativ zu diesem Feld bestimmt werden.

Durch zweifache Integration der durch einen Sensor in oder an einer Bilderfassungsvorrichtung erfassten linearen oder translatorischen Beschleunigung und durch einfache Integration der Drehrate können ausgehend von einer anfänglichen Position und einer anfänglichen Orientierung die Position und die Orientierung der Blickrichtung der Bilderfassungsvorrichtung berechnet werden.

Bei einem System, wie es hier beschrieben ist, ist der Signaleingang der Steuerung insbesondere zum Empfangen eines Bildsignals von einer Kamera zum Erfassen eines Operationsfelds ausgebildet, wobei die Steuerung zum Bestimmen der Orientierung der Bilderfassungsvorrichtung durch Auswertung eines am Signaleingang empfangenen Bildsignals vorgesehen und ausgebildet ist.

Ein System, wie es hier beschrieben ist, umfasst insbesondere ferner eine Kamera zum Erfassen eines Operationsfelds und zum Erzeugen eines Bildsignals, die mit dem Signaleingang der Steuerung gekoppelt ist.

Die Kamera zum Erfassen des Operationsfelds ist nicht mit der Bilderfassungsvorrichtung zu verwechseln. Die Kamera zum Erfassen des Operationsfelds ist insbesondere über oder schräg über oder neben dem Operationsfeld angeordnet. Die Kamera kann eine Stereokamera sein, so dass durch Auswertung ihres Stereobilds Information über die Orientierung und den Ort (in allen drei Dimensionen) der Bilderfassungsvorrichtung gewonnen werden kann. Die Kamera kann zur Erfassung eines Bilds in dem für das gesunde menschliche Auge sichtbaren Wellenlängenbereich oder einem Teil davon und/oder in einem oder mehreren anderen Wellenlängenbereichen (beispielsweise Infrarot) vorgesehen und ausgebildet sein.

Die Steuerung kann zum Empfangen und Auswerten von Bildsignalen von mehreren Kameras vorgesehen sein. Die Steuerung ist insbesondere zum Erkennen eines proximalen Endes einer Bilderfassungsvorrichtung oder einer besonderen Markierung an dem proximalen Ende einer Bilderfassungsvorrichtung vorgesehen und ausgebildet. Durch Erfassung der Position und optional auch der Größe der Abbildung des proximalen Endes der Bilderfassungsvorrichtung und ggf. Vergleich der Bilder mehrerer Kameras können die Position und die Orientierung des proximalen Endes der Bilderfassungsvorrichtung bestimmt werden. Aus der Position und Orientierung des proximalen Endes einer starren Bilderfassungsvorrichtung können die Position und Orientierung des distalen Endes der Bilderfassungsvorrichtung und insbesondere die Orientierung der Blickrichtung der Bilderfassungsvorrichtung berechnet werden.

Bei einem System, wie es hier beschrieben ist, ist der Signaleingang der Steuerung insbesondere zum Empfangen eines Signals von einem Empfänger für elektromagnetische Wellen, die von der Bilderfassungsvorrichtung ausgehen, ausgebildet, wobei die Steuerung zum Bestimmen der Orientierung der Bilderfassungsvorrichtung im Raum durch Auswertung des von dem Empfänger erhaltenen Signals vorgesehen und ausgebildet ist.

Ein System, wie es hier beschrieben ist, umfasst insbesondere ferner einen Empfänger zum Empfangen elektromagnetischer Wellen, die von der Bilderfassungsvorrichtung ausgehen.

Die Bilderfassungsvorrichtung kann einen oder mehrere Sensoren zur Erfassung der Orientierung der Bilderfassungsvorrichtung oder der Blickrichtung der Bilderfassungsvorrichtung und einen Sender zum Emittieren einer elektromagnetischen Welle oder einen Transponder zum Remittieren einer modifizierten elektromagnetischen Welle aufweisen. Der Sender oder Transponder ist so ausgebildet, dass die emittierte elektromagnetische Welle bzw. die modifiziert remittierte elektromagnetische Welle Information über die Orientierung und optional auch über die Position der Bilderfassungsvorrichtung enthält.

Alternativ kann die Bilderfassungsvorrichtung einen oder mehrere Reflektoren zum Reflektieren von elektromagnetischen Wellen und/oder einen oder mehrere Sender zum Emittieren elektromagnetischer Wellen aufweisen, wobei die Steuerung ausgebildet ist, um aus der Intensität oder Amplitude oder Leistungsdichte und/oder aus der Polarisation und/oder aus der Phasenlage von einem oder mehreren von der Bilderfassungsvorrichtung reflektierten oder emittierten und von dem Empfänger empfangenen elektromagnetischen Wellen auf die Orientierung der Bilderfassungsvorrichtung zu schließen.

Der Empfänger umfasst insbesondere eine Antenne oder mehrere Antennen zum Empfangen der elektromagnetischen Welle. Die Frequenz der elektromagnetischen Welle liegt insbesondere zwischen einigen hundert kHz und wenigen GHz.

Bei einem System, wie es hier beschrieben ist, ist die Steuerung insbesondere ausgebildet, um die Antriebseinrichtung so zu steuern, dass innerhalb eines vorbestimmten Winkelbereichs eine Rotation der Bilderfassungsvorrichtung um deren Blickrichtung um einen ersten Winkel eine Rotation der Bildwiedergabevorrichtung um einen zweiten Winkel bewirkt, wobei der zweite Winkel dem ersten Winkel gleicht oder ein vorbestimmter Bruchteil des ersten Winkels oder eine vorbestimmte nicht-konstante Funktion des ersten Winkels oder eine vorbestimmte Funktion der Winkelposition der Bilderfassungsvorrichtung ist.

Alternativ kann die Steuerung so ausgebildet sein, dass der zweite Winkel dem ersten Winkel gleicht.

Die Steuerung ist insbesondere ausgebildet, um die Antriebseinrichtung so zu steuern, dass innerhalb eines vorbestimmten Winkelbereichs jede Rotation der Bilderfassungsvorrichtung um deren Blickrichtung um einen ersten Winkel eine Rotation der Bildwiedergabevorrichtung um einen zweiten Winkel bewirkt.

Die Steuerung ist insbesondere ausgebildet, um die Antriebseinrichtung so zu steuern, dass innerhalb eines vorbestimmten Winkelbereichs eine oder jede Rotation der Bilderfassungsvorrichtung um deren Blickrichtung eine Rotation der Bildwiedergabevorrichtung um die mittlere Flächennormale der Bildwiedergabefläche der Bildwiedergabevorrichtung bewirkt. Die Steuerung ist insbesondere ausgebildet, um die Antriebseinrichtung so zu steuern, dass eine Rotation der Bilderfassungsvorrichtung in einer Rotationsrichtung (bezogen auf die Blickrichtung von der Bilderfassungsvorrichtung zu dem betrachteten Objekt) eine Rotation der Bildwiedergabevorrichtung in der gleichen Rotationsrichtung (bezogen auf die Richtung von einem Betrachter zu der Bildwiedergabevorrichtung) bewirkt.

Der vorbestimmte Winkelbereich kann 360 Grad, also einen Vollkreis, umfassen. Die Steuerung ist insbesondere so ausgebildet, dass der zweite Winkel jederzeit dem ersten Winkel oder einem vorbestimmten Bruchteil des ersten Winkels entspricht. Die Steuerung kann ferner so ausgebildet sein, dass Begrenzungen oder Randbedingungen seitens der Bildwiedergabevorrichtung, beispielsweise eine begrenzte Länge oder Elastizität eines Kabels zum Übertragen eines Bildsignals oder von elektrischer Leistung oder ein begrenzter Winkelbereich eines Gelenks der Haltevorrichtung berücksichtigt werden.

Bei einem System, wie es hier beschrieben ist, ist die Steuerung insbesondere ausgebildet, um die Antriebseinrichtung so zu steuern, dass innerhalb eines vorbestimmten Winkelbereichs eine oder jede Rotation einer mit der Bilderfassungsvorrichtung gekoppelten Kamera relativ zu der Bilderfassungsvorrichtung um die Blickrichtung der Kamera um einen ersten Winkel eine Rotation der Bildwiedergabevorrichtung um einen zweiten Winkel bewirkt, wobei der zweite Winkel dem ersten Winkel gleicht oder ein vorbestimmter Bruchteil des ersten Winkels oder eine vorbestimmte Funktion des ersten Winkels oder eine vorbestimmte Funktion der Winkelposition der Bilderfassungsvorrichtung ist.

Beispielsweise umfassen viele Endoskope selbst keine Kamera, sondern lediglich ein Okular, an das eine Kamera optisch und mechanisch gekoppelt werden kann. Eine Rotation der Kamera relativ zu dem Endoskop (oder zu einer anderen Bilderfassungsvorrichtung) in einer Rotationsrichtung bewirkt eine Rotation des durch das Bildsignal der Kamera repräsentierten Bilds in der entgegengesetzten Rotationsrichtung. Diese Rotation kann durch eine entsprechende Rotation der Bildwiedergabevorrichtung kompensiert werden. Alternativ kann die Rotation der Kamera relativ zu der Bilderfassungsvorrichtung durch eine Bearbeitung des von der Kamera erzeugten Bildsignals erfolgen, wobei das durch das Bildsignal repräsentierte Bild rotiert wird.

Grundsätzlich kann die Funktion, die die Orientierung der Bildwiedergabevorrichtung im Raum festlegt, bei einem hier beschriebenen System zusätzlich auch noch andere Faktoren berücksichtigen. So zum Beispiel die Position und/oder Orientierung des Anwenders relativ zum Operationsfeld und/oder relativ zur Bildwiedergabevorrichtung. Dazu können Mittel vorgesehen sein, die geeignet sind, die Position oder Orientierung einer Person zu erfassen, wie beispielsweise eine Markierung, die an der Person befestigt ist und von einer Kamera erfasst wird, oder Mittel zur Erkennung der Augen des Anwenders und deren Blickrichtung. Auf diese Weise kann verhindert werden, dass die Bildwiedergabevorrichtung für einen Anwender schlecht oder gar nicht mehr einsehbar ist, und eine Positionierung der Bildwiedergabevorrichtung relativ zum Anwender kann optimiert werden.

Bei einem System, wie es hier beschrieben ist, weist die Haltevorrichtung insbesondere ein oder mehrere Gelenke auf, die eine Rotation der Bildwiedergabevorrichtung um eine mittlere Flächennormale einer Bildwiedergabefläche der Bildwiedergabevorrichtung erlauben, wobei die Antriebseinrichtung einen oder mehrere Motoren oder Aktoren zum Rotieren der Bildwiedergabevorrichtung um die mittlere Flächennormale der Bildwiedergabefläche der Bildwiedergabevorrichtung umfasst.

Die Haltevorrichtung kann ein einziges Gelenk in Gestalt eines Rotationslagers aufweisen, das eine Rotation der Bildwiedergabevorrichtung um die mittlere Flächennormale der Bildwiedergabefläche einer in der vorgesehenen Weise mit der Haltevorrichtung mechanisch verbundenen Bildwiedergabevorrichtung ermöglicht. In diesem Fall weist die Antriebseinrichtung insbesondere auch nur einen Motor oder einen Aktor zur Rotation der in der vorgesehenen Weise mit der Haltevorrichtung mechanisch verbundenen Bildwiedergabevorrichtung auf. Alternativ kann die Haltevorrichtung beispielsweise als Hexapod ausgebildet sein oder einen oder mehrere Hexapoden umfassen. Eine Rotation der Bildwiedergabevorrichtung erfolgt in diesem Fall insbesondere durch Verkürzen mehrerer Arme und/oder gleichzeitiges Verlängern anderer Arme durch je einen Motor oder Aktor.

Ein System, wie es hier beschrieben ist, umfasst insbesondere ferner eine Bildsignalverarbeitungsvorrichtung mit einem Bildsignaleingang zum Empfangen eines ersten Bildsignals von der Bilderfassungsvorrichtung und einem Bildsignalausgang zum Bereitstellen eines zweiten Bildsignals für die Bildwiedergabevorrichtung, wobei die Bildsignalverarbeitungsvorrichtung ausgebildet ist, um das von der Bildwiedergabevorrichtung wiedergegebene Bild zu rotieren, wenn die Bilderfassungsvorrichtung rotiert wird.

Dazu umfasst die Bildsignalverarbeitungsvorrichtung insbesondere einen Signaleingang zum Empfangen eines Signals, das eine Orientierung oder eine Änderung der Orientierung der Bilderfassungsvorrichtung im Raum repräsentiert oder eine Bestimmung der Orientierung oder der Änderung der Orientierung der Bilderfassungsvorrichtung ermöglicht. Alternativ kann die Bildsignalverarbeitungsvorrichtung ausgebildet sein, um durch eine Analyse des von der Bilderfassungsvorrichtung erfassten Bilds, d. h. des von der Bilderfassungsvorrichtung oder deren Kamera erzeugten Bildsignals, eine Rotation der Bilderfassungsvorrichtung zu erkennen und eine entsprechende Rotation des durch das Bildsignal, das der Bildwiedergabevorrichtung zugeführt wird, repräsentierten Bilds zu bewirken.

Die Bildsignalverarbeitungsvorrichtung kann mit der Steuerung und/oder mit anderen Komponenten des Systems integriert sein.

Eine Steuerung für ein System, wie es hier beschrieben ist, umfasst insbesondere einen Signaleingang zum Empfangen eines Signals, das eine Orientierung oder eine Änderung der Orientierung der Blickrichtung der Bilderfassungsvorrichtung im Raum repräsentiert oder eine Bestimmung der Orientierung oder der Änderung der Orientierung der Blickrichtung der Bilderfassungsvorrichtung ermöglicht, und einen Steuersignalausgang, der mit dem Steuersignaleingang der steuerbaren Antriebseinrichtung koppelbar ist, zum Bereitstellen eines Steuersignals zum Steuern der steuerbaren Antriebseinrichtung, wobei die Steuerung vorgesehen und ausgebildet ist, um die steuerbare Antriebseinrichtung so zu steuern, dass innerhalb eines vorbestimmten Bereichs möglicher Orientierungen der Blickrichtung der Bilderfassungsvorrichtung im Raum die Orientierung der Bildwiedergabevorrichtung im Raum eine vorbestimmte Funktion der Orientierung der Blickrichtung der Bilderfassungsvorrichtung im Raum ist
Die Steuerung kann weitere Merkmale der Steuerung eines hier beschriebenen Systems aufweisen.

Ein Verfahren zum Wiedergeben eines mittels einer bewegbaren Bilderfassungsvorrichtung erfassten Bilds umfasst einen Schritt des Erfassens der Orientierung der Blickrichtung der Bilderfassungsvorrichtung im Raum mittels eines Sensors und einen Schritt des Einstellens der Orientierung der Bildwiedergabevorrichtung im Raum abhängig von der Orientierung der Bilderfassungsvorrichtung im Raum.

Das Verfahren ist insbesondere mit einem System, wie es hier beschrieben ist ausführbar. Ein System, wie es hier beschrieben ist, ist insbesondere für ein Verfahren, wie es hier beschrieben ist ausgebildet. Eine Steuerung, wie sie hier beschrieben ist, ist insbesondere zur Steuerung eines Verfahrens, wie es hier beschrieben ist, ausgebildet.

Beim Erfassen der Orientierung der Bilderfassungsvorrichtung im Raum werden insbesondere die Richtung der Schwerkraft und/oder eine lineare Beschleunigung und/oder eine Rotation (beispielsweise mittels eines Gyroskops, eines Vibrationskreisels oder eines anderen Drehratensensors) und/oder eine Richtung und/oder eine Stärke eines Magnetfelds erfasst. Der Sensor ist insbesondere Teil der Bilderfassungsvorrichtung oder mit dieser mechanisch starr (zerstörungsfrei lösbar oder nicht zerstörungsfrei lösbar) verbunden. Ein von dem Sensor erzeugtes Signal kann elektrisch, optisch oder mittels einer elektromagnetischen Welle zu einer Steuerung zum Steuern des Verfahrens übertragen werden.

Alternativ oder zusätzlich können eine oder mehrere Bilder eines Operationsfelds erfasst werden, um in dem Bild oder in den Bildern die Bilderfassungsvorrichtung zu erkennen und daraus die Orientierung und die Position der Bilderfassungsvorrichtung zu bestimmen. Alternativ oder zusätzlich können mittels einer oder mehrerer Antennen oder anderer Empfänger elektromagnetische Wellen, die von der Bilderfassungsvorrichtung erzeugt oder reflektiert oder remittiert werden, erfasst werden. Aus der Intensität oder Amplitude oder Leistungsdichte und/oder aus der Polarisation und/oder aus der Phasendifferenz mehrerer Wellen kann auf die Orientierung der Bilderfassungsvorrichtung geschlossen werden.

Bei einem Verfahren, wie es hier beschrieben ist, wird insbesondere ferner die Orientierung der Bilderfassungsvorrichtung im Raum an einer Bildwiedergabefläche der Bildwiedergabevorrichtung graphisch oder numerisch dargestellt.

Die Orientierung der Bilderfassungsvorrichtung im Raum kann beispielsweise durch eine perspektivische Darstellung eines Pfeils, der eine Grundrichtung andeutet, oder durch eine verkleinerte perspektivische Darstellung eines menschlichen Körpers in der Position des Patienten dargestellt werden. Alternativ oder zusätzlich können Winkel analog an Skalen oder digital in Gestalt von Zahlen angegeben werden.

Ein Verfahren, wie es hier beschrieben ist, umfasst insbesondere ferner ein Rotieren des auf der Bildwiedergabefläche dargestellten Bilds abhängig von einer Rotation der Bilderfassungsvorrichtung um die Blickrichtung der Bilderfassungsvorrichtung.

Eine Rotation der Bilderfassungsvorrichtung um die Blickrichtung der Bilderfassungsvorrichtung kann also alternativ oder gleichzeitig eine Rotation des auf der Bildwiedergabefläche der Bildwiedergabevorrichtung dargestellten Bilds relativ zu der Bildwiedergabevorrichtung und eine Rotation der Bildwiedergabevorrichtung bewirken.

Bei einem Verfahren, wie es hier beschrieben ist, wird das auf der Bildwiedergabefläche dargestellte Bild insbesondere abhängig von einer Orientierung der Blickrichtung der Bilderfassungsvorrichtung im Raum verformt.

Die Verformung erfolgt insbesondere so, dass ein perspektivischer Eindruck eines gegenüber der tatsächlichen Bildwiedergabefläche geschwenkten oder geneigten Bilds entsteht. Beispielsweise indem ein Schwenken oder Neigen der Bilderfassungsvorrichtung nur zum Teil ein entsprechendes Schwenken oder Neigen der Bildwiedergabevorrichtung, jedoch zusätzlich eine entsprechende Verformung des dargestellten Bilds bewirkt, kann auch ein größerer Schwenk- oder Neigewinkel dargestellt werden, ohne eine ungünstige Betrachtungsrichtung auf die Bildwiedergabefläche der Bilderfassungsvorrichtung zu bewirken.

### Kurzbeschreibung der Figuren

Nachfolgend werden Ausführungsformen anhand der beigefügten Figuren näher erläutert. Es zeigen:
- Figur 1: eine schematische Darstellung eines Systems zum Halten einer Bildwiedergabevorrichtung;
- Figur 2: eine schematische Darstellung eines weiteren Systems zum Halten einer Bildwiedergabevorrichtung;
- Figur 3: eine schematische Darstellung eines weiteren Systems zum Halten einer Bildwiedergabevorrichtung;
- Figur 4: eine schematische Darstellung eines weiteren Systems zum Halten einer Bildwiedergabevorrichtung;
- Figur 5: eine schematische Darstellung eines weiteren Systems zum Halten einer Bildwiedergabevorrichtung;
- Figur 6: eine schematische Darstellung einer Bilderfassungsvorrichtung;
- Figur 7: eine schematische Darstellung einer Bildwiedergabevorrichtung;
- Figur 8: eine weitere schematische Darstellung der Bildwiedergabevorrichtung aus Figur 7;
- Figur 9: eine schematische Darstellung eines weiteren Systems zum Halten einer Bildwiedergabevorrichtung;
- Figur 10: eine schematische Darstellung eines weiteren Systems zum Halten einer Bildwiedergabevorrichtung;
- Figur 11: schematische Darstellungen einer Bildwiedergabevorrichtung;
- Figur 12: ein schematisches Flussdiagramm eines Verfahrens zum Wiedergeben eines Bilds.
- Figur 13: ein schematisches Flussdiagramm eines Verfahrens zum Wiedergeben eines Bilds.

### Beschreibung der Ausführungsformen

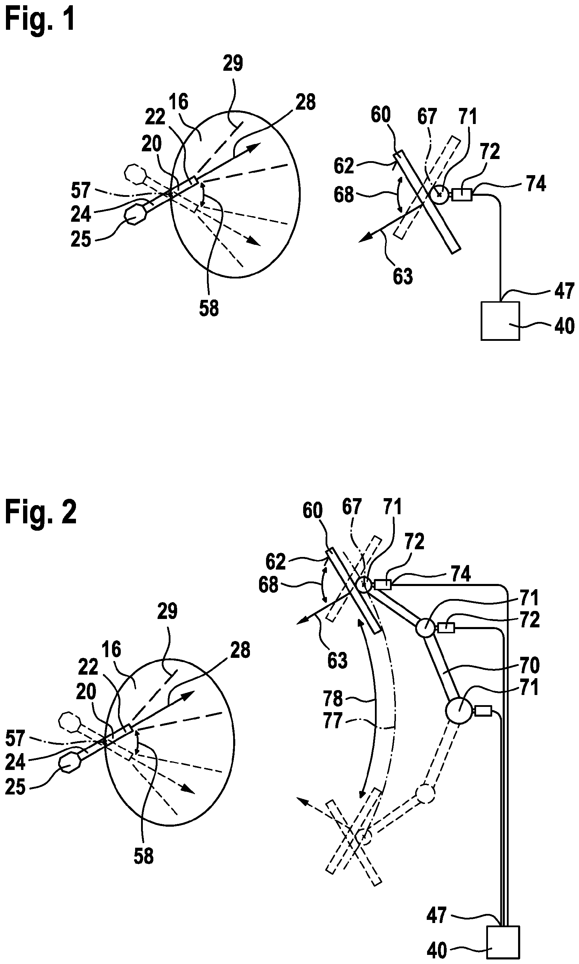

Figur 1 zeigt eine schematische Darstellung eines durch seine Kontur angedeuteten Hohlraums 16 im Körper eines Patienten. Ein distales Ende 22 einer Bilderfassungsvorrichtung, nämlich eines Endoskops 20, ist in den Hohlraum 16 eingeführt. Eine Kamera 25 ist mit dem proximalen Ende 24 des Endoskops 20 gekoppelt oder in das proximale Ende 24 des Endoskops 20 integriert. Alternativ kann das Endoskop 20 an seinem distalen Ende 22 oder an einem anderen Ort eine Kamera oder einen oder mehrere Bildsensoren aufweisen.

Die Blickrichtung 28 des Endoskops 20 ist die Richtung von einer Lichteintrittsfläche an dem distalen Ende 22 des Endoskops 20 zu einem weit entfernten Gegenstand, der in der Mitte eines von dem Endoskop 20 erfassten Bilds erscheint. Der Rand des von dem Endoskop 20 erfassten Bereichs ist in Figur 1 durch zwei Linien 29 angedeutet. Bei dem dargestellten Beispiel ist die Blickrichtung 28 des Endoskops 20 parallel zur Längsachse eines geraden Schafts des Endoskops 20.

Das Endoskop 20 kann - unter anderem - um eine Schwenk- oder Rotationsachse 57, die orthogonal zu der Zeichenebene der Figur 1 ist, rotiert oder geschwenkt werden. Die Schenk- oder Rotationsachse 57 liegt beispielsweise innerhalb eines Trokartubus, durch den das Endoskop in den Hohlraum 16 eingeführt ist.

Das Endoskop 20 ist in Figur 1 in zwei Orientierungen dargestellt, nämlich in durchgezogenen Linien in einer ersten Orientierung und in gestrichelten Linien in einer zweiten Orientierung. Das Endoskop 20 kann zwischen beiden Orientierungen (und weiteren Orientierungen) hin- und hergeschwenkt werden. Ein Doppelpfeil 58 deutet eine Schwenkbewegung um die Schwenk- oder Rotationsachse 57 an.

Das Endoskop 20 kann weitere Freiheitsgrade aufweisen, insbesondere ein Rotieren um seine Blickrichtung 28 und ein Rotieren oder Schwenken um eine weitere Rotations- bzw. Schwenkachse, die orthogonal zu der Schwenkachse 57 und orthogonal zu der Blickrichtung 28 des Endoskops 20 sein kann. Im Folgenden wird jedoch zunächst nur der Freiheitsgrad der Schwenkbewegung 58 um die Schwenk- bzw. Rotationsachse 57 betrachtet.

Durch in Figur 1 nicht dargestellte Vorrichtungen, insbesondere elektrische oder optische Signalleitungen und optional eine Bildsignalverarbeitungseinrichtung, wird ein mittels des Endoskops 20 (insbesondere durch die Kamera 25 oder einen oder mehrere Bildsensoren des Endoskops 20) erfasstes Bild zu einem Bildschirm 60 als Bildwiedergabevorrichtung übertragen. Das Bild wird an einer Bildwiedergabefläche 62 wiedergegeben. Die Bildwiedergabefläche 62 ist bei dem dargestellten Beispiel eben, und die Flächennormale 63 der Bildwiedergabefläche 62 ist parallel zu der Zeichenebene der Figur 1.

Der Bildschirm 60 ist an einem Gelenk 71, das eine Schwenk- bzw. Rotationsachse 67 definiert, gehalten. Der Bildschirm 60 ist also um die Schwenkachse 67 schwenkbar. Die Schwenkachse 67 ist orthogonal zu der Zeichenebene der Figur 1 und parallel zu der Bildwiedergabefläche 62 des Bildschirms 60.

Das Gelenk 71 ist Teil einer in Figur 1 im Übrigen nicht dargestellten bewegbaren Haltevorrichtung zum veränderbaren Halten des Bildschirms 60. Diese bewegbare Haltevorrichtung kann weitere Freiheitsgrade aufweisen, im Folgenden wird jedoch zunächst nur auf den durch das Gelenk 71 und die Rotations- bzw. Schwenkachse 67 definierten Freiheitsgrad des Schwenkens bzw. Rotierens 68 des Bildschirms um die Rotations- bzw. Schwenkachse 67 eingegangen.

An dem Gelenk 71 ist eine Antriebseinrichtung 72 zum Bewegen des Bildschirms 60, nämlich zum Schwenken des Bildschirms 60 um die Schwenkachse 67 vorgesehen. Ein Steuersignaleingang 74 der Antriebseinrichtung 72 ist mit einem Signalausgang 47 einer Steuerung 40 verbunden.

Die Steuerung 40 empfängt von einer in Figur 1 nicht dargestellten Einrichtung ein Signal, das eine Orientierung oder eine Änderung der Orientierung des Endoskops 20, insbesondere die momentane Blickrichtung 28 und/oder eine Änderung der Blickrichtung 28 repräsentiert oder eine Bestimmung der Orientierung oder der Änderung der Orientierung des Endoskops 20 ermöglicht. Wenn die Steuerung ein Signal empfängt, das eine Änderung der Orientierung des Endoskops 20 repräsentiert, bestimmt die Steuerung 40 durch Integration des Signals die gegenwärtige Orientierung des Endoskops 20. Wenn die Steuerung 40 ein Signal empfängt, das eine Bestimmung der Orientierung des Endoskops 20 ermöglicht, bestimmt die Steuerung 40 aus dem empfangenen Signal die Orientierung des Endoskops 20. Wenn die Steuerung 40 ein Signal empfängt, das die Bestimmung einer Änderung der Orientierung des Endoskops 20 ermöglicht, bestimmt die Steuerung 40 die Änderung der Orientierung des Endoskops 20 und integriert diese Änderung, um die gegenwärtige Orientierung des Endoskops 20 zu bestimmen. Die Änderung der Orientierung des Endoskops 20 kann beispielsweise für einen Freiheitsgrad in Grad pro Sekunde angegeben werden.

Die Steuerung 40 kennt insbesondere ferner die gegenwärtige Orientierung des Bildschirms 60 und vergleicht die Orientierung des Bildschirms 60 mit der durch das empfangene Signal repräsentierten Orientierung oder der durch die Steuerung 40 bestimmten Orientierung des Endoskops 20. Die Steuerung 40 erzeugt ein Steuersignal für die Antriebseinrichtung 72, um den Bildschirm 60 jederzeit in eine Orientierung zu bewegen, die der gegenwärtigen Orientierung des Endoskops 20 entspricht oder zugeordnet ist.

In Figur 1 ist der Bildschirm 60 in zwei Orientierungen dargestellt, nämlich in durchgezogenen Linien in einer ersten Orientierung, die der Orientierung des Endoskops 20, die in Figur 1 ebenfalls in durchgezogenen Linien dargestellt ist, entspricht, und in gestrichelten Linien in einer zweiten Orientierung, die der in Figur 1 ebenfalls in gestrichelten Linien dargestellten Orientierung des Endoskops 20 entspricht.

Bei dem in Figur 1 gezeigten Beispiel entspricht die Orientierung des Bildschirms 60 der Orientierung des Endoskops 20, wenn die Flächennormale 63 der Bildwiedergabefläche 62 des Bildschirms 60 entgegengesetzt parallel zu der Blickrichtung 28 des Endoskops 20 ist. Alternativ kann die Steuerung 40 die Antriebseinrichtung 72 beispielsweise so steuern, dass zwischen der Flächennormalen 63 der Bildwiedergabefläche 62 des Bildschirms 60 und der Blickrichtung 28 des Endoskops 20 jederzeit ein vorbestimmter Differenzwinkel liegt. Alternativ kann die Steuerung 40 die Antriebseinrichtung 72 beispielsweise so steuern, dass eine bei einer Initialisierung der Steuerung 40 oder zu einem anderen definierten Zeitpunkt vorliegende Orientierung des Bildschirms 60 einer zu dem gleichen Zeitpunkt vorliegenden Orientierung der Blickrichtung 28 des Endoskops 20 zugeordnet ist. In diesem Fall kann die Steuerung 40 die Antriebseinrichtung 72 so steuern, dass jede nachfolgende Änderung der Orientierung der Blickrichtung 28 des Endoskops 20 um einen bestimmten Winkel eine Änderung der Orientierung des Bildschirms 60 um den gleichen Winkel oder um einen vorbestimmten Bruchteil des Winkels oder um einen Winkel, der von der Abweichung der Orientierung des Bildschirms 60 von einer vorbestimmten Grundorientierung abhängt, bewirkt.

Die Schwenkachse 57, um die das Endoskop 20 schwenkbar ist, und die Schwenkachse 67, um die der Bildschirm 60 schwenkbar ist, sind bei dem in Figur 1 dargestellten Beispiel parallel zueinander und orthogonal zur Zeichenebene der Figur 1. Beide Schwenkachsen 57, 67 können beispielsweise vertikal, d. h. parallel zur Richtung der Gravitation, oder horizontal, d. h. orthogonal zur Vertikalen sein oder beliebige andere Richtungen aufweisen. Abweichend von der Darstellung in Figur 1 können die Schwenkachsen 57, 67 nicht-parallel sein.

Die durch das Gelenk 71 stellvertretend dargestellte Haltevorrichtung, die steuerbare Antriebseinrichtung 72 und die Steuerung 40 bilden ein System zum Halten des Bildschirms 60. Der Bildschirm 60 ist nicht Bestandteil dieses Systems, jedoch mit der durch das Gelenk 71 repräsentierten Haltevorrichtung mechanisch starr und zerstörungsfrei lösbar verbunden oder verbindbar. Alternativ kann der Bildschirm 60 Teil des Systems sein. Auch das Endoskop 20 ist nicht Teil des Systems, kann jedoch Teil des Systems sein.

Figur 2 zeigt eine schematische Darstellung eines Endoskops 20, dessen distales Ende 22 in einem Hohlraum 16 im Körper eines Patienten angeordnet ist, eines Bildschirms 60 und eines Systems aus einer bewegbaren Haltevorrichtung 70, einer mehrteiligen steuerbaren Antriebseinrichtung 72 und einer Steuerung 40. Das Endoskop 20 und der Bildschirm 60 entsprechen insbesondere dem Endoskop 20 und dem Bildschirm 60, der anhand der Figur 1 dargestellten Ausführungsform. Die Steuerung 40 und die bewegbare Haltevorrichtung 70 mit der mehrteiligen steuerbaren Antriebseinrichtung 72 ähneln in einigen Merkmalen, Eigenschaften und Funktionen der Steuerung 40 und der durch das Gelenk 71 repräsentierten Haltevorrichtung, die anhand der Figur 1 dargestellt wurden. Nachfolgend sind insbesondere Merkmale, Eigenschaften und Funktionen der Steuerung 40, der Haltevorrichtung 70 und der mehrteiligen Antriebseinrichtung 72 beschrieben, in denen diese sich von dem anhand der Figur 1 dargestellten Beispiel unterscheiden.

Die in Figur 2 gezeigte Haltevorrichtung 70 weist mehrere Gelenke 71 auf, die mehrere Schwenkachsen definieren. Diese Schwenkachsen können orthogonal zur Zeichenebene der Figur 2 sein und/oder eine oder mehrere andere Richtungen aufweisen. Bei dem dargestellten Beispiel definiert das dem Bildschirm 60 nächstgelegene Gelenk 71 eine Schwenkachse 67 orthogonal zu der Zeichenebene der Figur 2. Jedem Gelenk 71 ist ein Teil der mehrteiligen Antriebseinrichtung 72 zugeordnet. Jeder Teil der Antriebseinrichtung 72 ist mit einem Signalausgang 47 der Steuerung 40 gekoppelt.

Die Steuerung 40 ist ausgebildet, um die mehrteilige Antriebseinrichtung 72 so zu steuern, dass jede Schwenkbewegung des Endoskops 20 um die Schwenkachse 57 eine zugeordnete Schwenkbewegung des Bildschirms 60 um die Schwenkachse 67 und gleichzeitig eine translatorische Bewegung 78 des Bildschirms 60 entlang eines gekrümmten, insbesondere kreisbogenförmigen Pfads 77 bewirkt. Die Steuerung 40 ist insbesondere ausgebildet, um die mehrteilige Antriebseinrichtung 72 so zu steuern, dass in jeder Orientierung und Position des Bildschirms 60 die Flächennormale 63 der Bildwiedergabefläche 62 des Bildschirms 60 und der gekrümmte Pfad 77 in einer Ebene liegen. Zwei Orientierungen und Positionen des Bildschirms 60 sind in Figur 2 angedeutet, nämlich in durchgezogenen Linien eine Orientierung und eine Position des Bildschirms 60, die der in Figur 2 ebenfalls in durchgezogenen Linien dargestellten Orientierung des Endoskops 20 zugeordnet sind, und in gestrichelten Linien eine Orientierung und eine Position des Bildschirms 60, die der in Figur 2 in gestrichelten Linien angedeuteten Orientierung des Endoskops 20 zugeordnet sind.

Bei den anhand der Figuren 1 und 2 dargestellten Beispielen ist jeweils nur ein rotatorischer Freiheitsgrad der Schwenkbewegung 58 des Endoskops 20 um die Schwenk- oder Rotationsachse 57 und ein rotatorischer Freiheitsgrad der Schwenkbewegung 68 des Bildschirms 60 um die Schwenk- oder Rotationsachse 67 beschrieben. Im Fall des anhand der Figur 2 dargestellten Beispiels kommt ein translatorischer Freiheitsgrad der Bewegung 78 des Bildschirms 60 entlang des Pfads 77 hinzu, wobei die Steuerung 40 den rotatorischen Freiheitsgrad und den translatorischen Freiheitsgrad des Bildschirms 60 so miteinander verknüpft, dass jeder Orientierung des Bildschirms 60 eine bestimmte Position des Bildschirms 60 zugeordnet ist.

Zusätzlich zu den anhand der Figuren 1 und 2 dargestellten Freiheitsgraden kann die Steuerung einen oder mehrere weitere rotatorische Freiheitsgrade des Endoskops 20 und der Kamera 25 erfassen, nämlich eine Schwenkbewegung des Endoskops 20 um eine weitere Schwenkachse, die insbesondere zu der in den Figuren 1 und 2 dargestellte Schwenkachse 57 orthogonal ist, und/ oder eine Rotation des Endoskops 20 um die Blickrichtung 28 und/oder eine Rotation der Kamera 25 relativ zu dem Endoskop 20 beispielsweise um die optische Achse eines Okulars am proximalen Ende 24 des Endoskops 20 und eines Objektivs der Kamera 25.

Die Steuerung kann in diesem Fall ausgebildet sein, um den Bildschirm 60 abhängig von den Bewegungen des Endoskops 20 zusätzlich um eine oder mehrere weitere Schwenk- oder Rotationsachsen zu schwenken oder zu rotieren. Die Haltevorrichtung 70, die mehrteilige Antriebseinrichtung 72 und die Steuerung 40 können so ausgebildet sein, dass der Bildschirm 60 abhängig von der Blickrichtung 28 des Endoskops 20 um zwei zueinander orthogonale und zu der Flächennormale 63 der Bildwiedergabefläche 62 des Bildschirms 60 nicht parallele Schwenk- oder Rotationsachsen geschwenkt und optional gleichzeitig entlang einer gekrümmten Fläche bewegt wird. Dabei ist jeder Orientierung der Blickrichtung 28 des Endoskops 20 eine Orientierung und eine Position des Bildschirms 60 zugeordnet.

Alternativ oder zusätzlich kann die Haltevorrichtung 70 ein Gelenk, das eine Rotationsachse parallel zu der Flächennormale 63 der Bildwiedergabefläche 62 des Bildschirms 60 definiert, aufweisen und die Steuerung 40 so ausgebildet sein, dass eine Rotation des Endoskops 20 um seine Blickrichtung 28 und/oder eine Rotation der Kamera 25 relativ zu dem Endoskop 20 eine Rotation des Bildschirms 60 um die Flächennormale 63 der Bildwiedergabefläche 62 bewirkt.

Figur 3 zeigt eine schematische Darstellung einer Ärztin oder eines Arztes 12 mit einem medizinischen Instrument 14, beispielsweise einer Zange oder einer Schere. Das distale Ende des medizinischen Instruments 14 ist in einem Hohlraum 16 im Körpers eines Patienten angeordnet, um eine medizinische Maßnahme an einem Organ oder einem anderen Objekt 18 in dem Körper des Patienten zu ermöglichen. Das distale Ende 22 eines Endoskops 20 ist ebenfalls in dem Hohlraum 16 im Körper des Patienten angeordnet. Die Blickrichtung 28 des Endoskops 20 ist auf das distale Ende des Instruments 14 und auf das Organ 18 gerichtet.

Mittels des Endoskops 20 wird ein Bild des distalen Endes des medizinischen Instruments 14 und des Organs 18 in dem Hohlraum 16 in dem Körper des Patienten erfasst. Dazu erzeugen ein oder mehrere Bildsensoren des Endoskops 20 ein Bildsignal, das das erfasste Bild repräsentiert. Alternativ ist ähnlich wie bei den anhand der Figuren 1 und 2 dargestellten Beispielen an dem proximalen Ende 24 des Endoskops 20 eine Kamera angeordnet, die beispielsweise mit einem Okular des Endoskops 20 gekoppelt ist, ein durch das Endoskop 20 erzeugtes Bild erfasst und ein das Bild repräsentiertes Bildsignal erzeugt.

Gegenüber dem medizinischen Personal 12 ist ein Bildschirm 60 so angeordnet, dass das medizinische Personal 12 die Bildwiedergabefläche des Bildschirms 60 in einer möglichst wenig ermüdenden Haltung betrachten kann. Der Bildschirm 60 erhält das mittels des Endoskops 20 (das heißt durch das Endoskop selbst oder durch eine mit dem Endoskop optisch gekoppelte Kamera) erzeugte Bildsignal und gibt das durch das Bildsignal repräsentierte Bild wieder.

Der Bildschirm 60 wird von einer bewegbaren Haltevorrichtung 70, die - ähnlich wie anhand der Figuren 1 und 2 dargestellt - ein oder mehrere Gelenke, die in Figur 3 nicht dargestellt sind, aufweist, gehalten. Eine ein- oder mehrteilige Antriebseinrichtung 72 zum Bewegen der Haltevorrichtung und des Bildschirms 60 ist mit einem Signalausgang 47 einer Steuerung 40 gekoppelt. Signaleingänge 42 der Steuerung 40 sind mit zwei oder mehr Kameras 30 an einem Stativ 32 gehalten. An dem proximalen Ende 24 des Endoskops 20 ist eine Struktur 26 angeordnet. Bei dem dargestellten Beispiel ist die Struktur 26 kreuzförmig. Die Struktur 26 macht eine optische Erkennung des proximalen Endes 24 des Endoskops 20 sowie seiner Position und Orientierung einfacher und/oder zuverlässiger.

Die Kameras 30 sind auf das Operationsfeld gerichtet und erfassen das proximale Ende 24 des Endoskops 20 mit der Struktur 26. Die Steuerung 40 erhält Bildsignale von den Kameras 30 und wertet diese aus, um darin die Struktur 26 zu erkennen und die Position und die Orientierung des proximalen Endes 24 des Endoskops 20 zu bestimmen. Abhängig von der durch die Steuerung 40 mittels der Kameras 30 bestimmten Position und Orientierung des proximalen Endes 24 des Endoskops 20 steuert die Steuerung 40 - durch ein Steuersignal an den Steuersignaleingang 74 der Antriebseinrichtung 72 - die Antriebseinrichtung 72 so, dass der Bildschirm 60 jederzeit eine Orientierung, die der gleichzeitig vorliegenden Orientierung des Endoskops 20, insbesondere der Blickrichtung 28 des Endoskops 20, entspricht oder zugeordnet ist, aufweist.

Die Steuerung 40, die Haltevorrichtung 70 und die Antriebseinrichtung 72 bilden ein System zum Halten des Bildschirms 60.

Figur 4 zeigt eine schematische Darstellung einer Ärztin oder eines Arztes 12 mit einem medizinischen Instrument 14, dessen distales Ende in einem Hohlraum 16 im Körper eines Patienten angeordnet ist. Das distale Ende 22 eines Endoskops 20 ist ebenfalls in dem Hohlraum 16 angeordnet. Die Blickrichtung 28 des Endoskops 20 ist auf ein Organ 18 und das distale Ende des medizinischen Instruments 14 gerichtet. Gegenüber dem medizinischen Personal 12 ist ein Bildschirm 60 an einer bewegbaren Haltevorrichtung 70 angeordnet, auf dem ein mittels des Endoskops 20 erfasstes Bild wiedergegeben wird. Ein Signalausgang 47 einer Steuerung 40 ist mit einem Steuersignaleingang 74 einer Antriebseinrichtung 72 an der bewegbaren Haltevorrichtung 70 gekoppelt, so dass die Steuerung 40 die Antriebseinrichtung 72 steuern kann.

Das Endoskop 20, die Steuerung 40, der Bildschirm 60, die bewegbare Haltevorrichtung 70 und die Antriebseinrichtung 72 ähneln in einigen Merkmalen, Eigenschaften und Funktionen dem Endoskop 20, der Steuerung 40, dem Bildschirm 60, der bewegbaren Haltevorrichtung 70 und der Antriebseinrichtung 72, die anhand der Figur 3 dargestellt sind. Insbesondere bilden die Steuerung 40, die Haltevorrichtung 70 und die Antriebseinrichtung 72 ein System zum Halten des Bildschirms 60. Nachfolgend sind insbesondere Merkmale, Eigenschaften und Funktionen beschrieben, in denen das Endoskop 20 und die Steuerung 40 sich von dem anhand der Figur 3 dargestellten unterscheiden.

Das Endoskop 20 weist an seinem proximalen Ende 24 einen Transponder 36 zum Empfangen einer elektromagnetischen Welle und zum Senden einer abhängig von der Orientierung des Endoskops 20 und des Transponders 36 modifizierte elektromagnetische Welle auf. Ein Signaleingang 42 der Steuerung 40 ist mit einer Einrichtung 34 zum Senden eines elektromagnetischen Signals und zum Empfangen eines von dem Transponder 36 ausgehenden elektromagnetischen Signals gekoppelt.

Der Transponder 36 an dem Endoskop 20 kann ausgebildet sein, um selbst die Orientierung des Endoskops 20 zu erfassen und Information über die Orientierung des Endoskops 20 in ein an die Einrichtung 34 zurückgesandtes elektromagnetisches Signal zu codieren. Alternativ kann der Transponder 36 als rein passives Bauelement ausgebildet sein, wobei die Einrichtung 34 und/ oder die Steuerung 40 die Orientierung des Transponders 36 und des Endoskops 20 beispielsweise aus einer Polarisationsrichtung, einer Leistungsdichte oder einer Phasenlage eines von dem Transponder 36 zu der Einrichtung 34 übertragenen elektromagnetischen Signals ableiten oder bestimmen. Sowohl die Einrichtung 34 als auch der Transponder 36 können jeweils eine oder mehrere Antennen für unterschiedliche Polarisationen und/oder Abstrahlcharakteristika und/oder für elektromagnetische Wellen mit unterschiedlichen Phasenlagen aufweisen.

Während einer medizinischen Maßnahme erzeugt die Einrichtung 34 eine elektromagnetische Welle, die von dem Transponder 36 an dem Endoskop 20 reflektiert oder remittiert wird. Die Einrichtung 34 empfängt die von dem Transponder 36 reflektierte oder remittierte elektromagnetische Welle. Dabei empfängt die Einrichtung 34 insbesondere die in der zurückgesandten elektromagnetischen Welle codierte Information und/oder die Intensität oder Amplitude oder Leistungsdichte der empfangenen elektromagnetischen Welle und/oder die Polarisation der elektromagnetischen Welle und/oder die Phasenlage der elektromagnetischen Welle. Wenn der Transponder 36 mehrere Antennen aufweist oder mehrere Teiltransponder umfasst, kann die Einrichtung 34 die Amplituden, Polarisationen und/oder Phasenlagen der von diesen Antennen oder Teiltranspondern ausgehenden elektromagnetischen Wellen absolut oder relativ zueinander erfassen.

Die Einrichtung 34 kann ausgebildet sein, um aus den empfangenen elektromagnetischen Wellen die Orientierung des Transponders 36 und des Endoskops 20 zu bestimmen und diese Information an die Steuerung 40 zu übertragen. Alternativ kann die Einrichtung 34 ausgebildet sein, um Rohdaten an die Steuerung 40 zu übertragen, wobei die Orientierung des Transponders 36 und des Endoskops 20 erst durch die Steuerung 40 aus den Rohdaten bestimmt wird.

Alternativ zu einem Transponder 36 kann das Endoskop 20 mit einem Sender oder mit mehreren Sendern versehen sein, der oder die über eine oder mehrere Antennen eine oder mehrere elektromagnetische Wellen senden. Dabei empfängt die Einrichtung 34 insbesondere die in der von dem Sender oder den Sendern gesendeten elektromagnetischen Wellen codierte Information und/ oder die Intensität oder Amplitude oder Leistungsdichte der empfangenen elektromagnetischen Welle und/oder die Polarisation der elektromagnetischen Welle und/oder die Phasenlage der elektromagnetischen Welle. Wenn der Sender mehrere Antennen aufweist oder mehrere Sender vorgesehen sind, kann die Einrichtung 34 die Amplituden, Polarisationen und/oder Phasenlagen der von diesen Antennen oder Sendern ausgehenden elektromagnetischen Wellen absolut oder relativ zueinander erfassen.

Ähnlich wie anhand der Figuren 1 bis 3 dargestellt, steuert die Steuerung 40 die Antriebseinrichtung 72 derart, dass die Orientierung des Bildschirms 60 jederzeit einer Soll-Orientierung des Bildschirms 60 entspricht, die der gleichzeitig vorliegenden Orientierung des Endoskops 20 zugeordnet ist.

Figur 5 zeigt eine schematische Darstellung einer Ärztin oder eines Arztes 12 mit einem medizinischen Instrument 14. Das distale Ende des medizinischen Instruments 14 und das distale Ende 22 eines Endoskops 20 sind für eine medizinische Maßnahme in einem Hohlraum 16 im Körper eines Patienten angeordnet. Die Blickrichtung 28 des Endoskops 20 ist auf das distale Ende des Instruments 14 und ein Organ 18 in dem Hohlraum 16 gerichtet. Das proximale Ende 24 des Endoskops 20 ist außerhalb des Hohlraums 16 angeordnet.

Das Endoskop 20, die Steuerung 40, der Bildschirm 60, die bewegbare Haltevorrichtung 70 und die Antriebseinrichtung 72 ähneln in einigen Merkmalen, Eigenschaften und Funktionen den Endoskopen 20, den Steuerungen 40, den Bildschirmen 60, den bewegbaren Haltevorrichtungen 70 und den Antriebseinrichtungen 72, die anhand der Figuren 3 und 4 dargestellt sind. Insbesondere bilden die Steuerung 40, die Haltevorrichtung 70 und die Antriebseinrichtung 72 ein System zum Halten des Bildschirms 60. Nachfolgend sind insbesondere Merkmale, Eigenschaften und Funktionen beschrieben, in denen das Endoskop 20 und die Steuerung 40 sich von dem anhand der Figur 3 dargestellten unterscheiden.

Das Endoskop 20 weist in oder an seinem proximalen Ende 24 einen Sensor 38 auf. Der Sensor 38 ist vorgesehen und ausgebildet zum Erfassen der Richtung der Schwerkraft und/oder zum Erfassen einer linearen oder translatorischen Beschleunigung und/oder zum Erfassen einer Drehrate und/oder zum Erfassen der Richtung und/oder der Stärke eines Magnetfelds oder eines anderen gerichteten Felds. Der Sensor 38 ist mit einem Signaleingang 42 der Steuerung 40 gekoppelt. Die Steuerung 40 erhält von dem Sensor 38 die erfasste Information und bestimmt daraus die Orientierung des Sensors 38 und des Endoskops 20 im Raum. Alternativ bestimmt der Sensor 38 oder eine dem Sensor nachgeschaltete Einrichtung in oder an dem Endoskop 20 die Orientierung des Sensors 38 und des Endoskops 20 im Raum und überträgt diese Information an die Steuerung 40.

Ähnlich wie anhand der Figuren 1 bis 4 dargestellt, steuert die Steuerung 40 die Antriebseinrichtung 72 derart, dass die Orientierung des Bildschirms 60 jederzeit einer Soll-Orientierung des Bildschirms 60 entspricht, die der gleichzeitig vorliegenden Orientierung des Endoskops 20 zugeordnet.

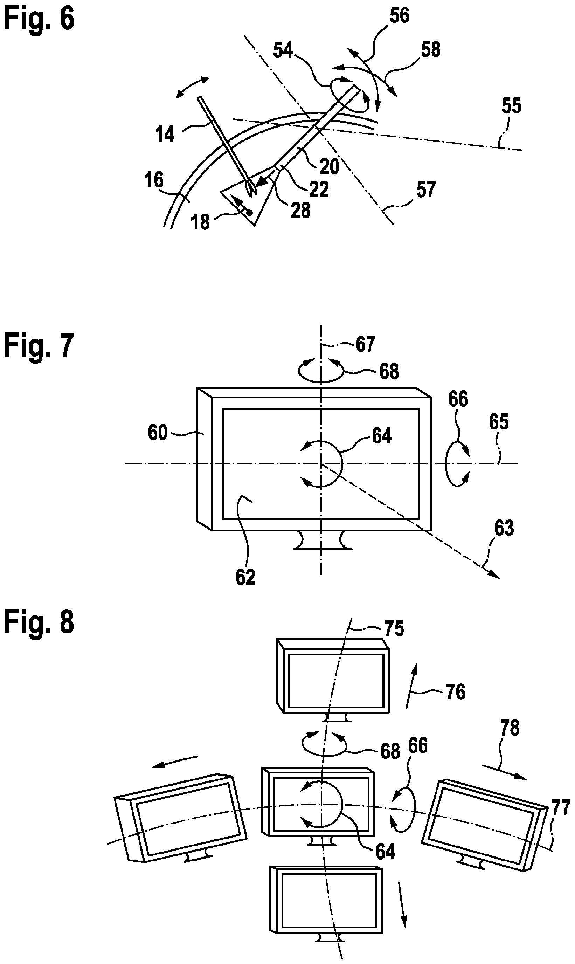

Figur 6 zeigt eine schematische Darstellung eines Endoskops 20, dessen distales Ende 22 innerhalb und dessen proximales Ende 24 außerhalb eines Hohlraums 16 im Körper eines Patienten angeordnet sind. Ferner sind ein medizinisches Instrument 14, dessen distales Ende in dem Hohlraum 16 angeordnet ist, und ein Organ 18 in dem Hohlraum 16 dargestellt. Das Organ wird durch einen Pfeil repräsentiert.

In Figur 6 sind Rotationsachsen und dazugehörige Bewegungen zu drei rotatorischen Freiheitsgraden dargestellt. Ein erster rotatorischer Freiheitsgrad wird durch die Rotation 54 um die Blickrichtung 28 des Endoskops 20 gebildet. Ferner kann das Endoskop 20 um zwei zu einander und zu der Blickrichtung 28 des Endoskops 20 orthogonale Rotations- oder Schwenkachsen 55, 57 rotiert oder geschwenkt werden, entsprechend zwei weiteren Freiheitsgraden. Die Schwenkachse 55 ist horizontal und orthogonal zu der Blickrichtung 28, die Schwenkachse 57 ist orthogonal zu der Blickrichtung 28 und zu der Schwenkachse 55. Alternativ kann die Schwenkachse 57 vertikal, d. h. parallel zur Richtung der Gravitation, sein. Die Schwenkbewegung um die horizontale Schwenkachse 55 ist durch einen Doppelpfeil 56 angedeutet. Die Schwenkbewegung um die Schwenkachse 57 ist durch einen Doppelpfeil 58 angedeutet.

Figur 7 zeigt eine schematische Darstellung eines Bildschirms, der Teil der anhand der Figuren 1 bis 5 dargestellten Systeme sein oder durch eines der anhand der Figuren 1 bis 5 dargestellten Systeme gehalten werden kann.

Der Bildschirm 60 weist drei rotatorische Freiheitsgrade auf. Die Rotation 64 um die Flächennormale 63 der Bildwiedergabefläche 62 des Bildschirms 60 wird als erster Freiheitsgrad bezeichnet. Schwenkbewegungen 66, 68 um zwei weitere Schwenk- oder Rotationsachsen 65, 67 entsprechen zwei weiteren Freiheitsgraden. Bei dem dargestellten Beispiel sind die Schwenkachsen 65, 67 zu der Flächennormalen 63 der Bildwiedergabefläche 62 des Bildschirms 60 und zueinander orthogonal. Jede Schwenkachse 65, 67 ist zu zwei geraden Randabschnitten der rechteckigen Bildwiedergabefläche 62 parallel.

Figur 8 zeigt eine weitere schematische Darstellung des Bildschirms 60 aus Figur 7 mit zusätzlichen translatorischen Freiheitsgraden. Der Bildschirm 60 ist entlang einer gekrümmten Fläche bewegbar, die durch einen in einer vertikalen Ebene liegenden Pfad 75 und einen in einer horizontalen Ebene liegenden Pfad 77 angedeutet ist. Pfeile 76 deuten Bewegungen des Bildschirms 60 entlang des Pfads 75 in einer vertikalen Ebene an, Pfeile 78 deuten Bewegungen des Bildschirms 60 entlang des Pfads 77 in einer horizontalen Ebene an. Der Bildschirm 60 ist in durchgezogenen Linien in einer Grundposition und in entlang der Pfade 75, 77 verschobenen Positionen angedeutet. Dabei ist auch angedeutet, dass - ähnlich wie anhand der Figur 2 dargestellt - mit einer Schwenkbewegung 66, 68 des Bildschirms 60 eine translatorische Bewegung 76, 78 einhergeht.

Die Steuerung 40 eines der anhand der Figuren 1 bis 5 dargestellten Systeme ist insbesondere so ausgebildet, dass eine Rotationsbewegung 54 des Endoskops 20 um seine Blickrichtung 28 (vgl. Figur 6) eine Rotationsbewegung 64 des Bildschirms 60 um die Flächennormale 63 der Bildwiedergabefläche 62 (vgl. Figur 7) bewirkt. Alternativ oder zusätzlich kann die Steuerung ausgebildet sein, um die Antriebseinrichtung so zu steuern, dass eine Schwenkbewegung 56 des Endoskops 20 um eine horizontale Schwenkachse 55 eine Schwenkbewegung 66 des Bildschirms 60 um eine horizontale Schwenkachse 65 und optional gleichzeitig eine Translationsbewegung 76 entlang eines Pfads 75 in einer vertikalen Ebene (vgl. Figur 8) bewirkt. Alternativ oder zusätzlich kann die Steuerung 40 ausgebildet sein, um die Antriebseinrichtung so zu steuern, dass eine Schwenkbewegung 58 des Endoskops 20 um eine vertikale oder im Wesentlichen vertikale Schwenkachse 57 oder um eine Schwenkachse 57, die zu der Blickrichtung 28 des Endoskops 20 und zu einer Horizontalen orthogonale ist, (vgl. Figur 6) eine Schwenkbewegung 68 des Bildschirms 60 um eine vertikale oder im Wesentlichen vertikale Achse 67 (vgl. Figur 7) und optional zusätzlich eine Translationsbewegung 78 entlang eines horizontalen Pfads 77 (vgl. Figur 8) bewirkt.

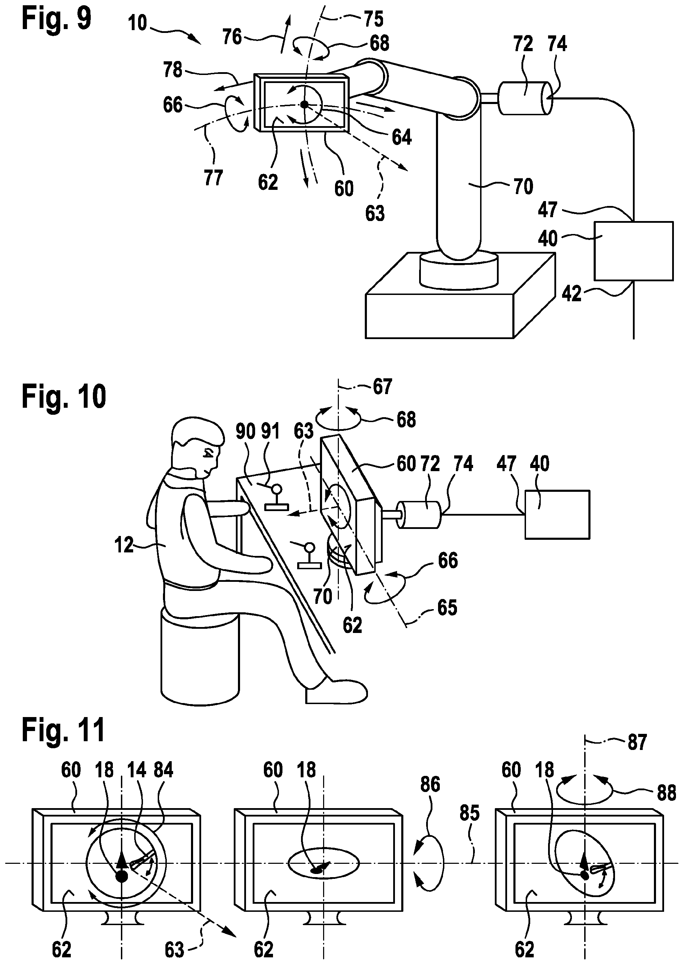

Figur 9 zeigt eine schematische Darstellung eines weiteren Systems aus einer Steuerung 40 mit einem Signaleingang 42 und einem Signalausgang 47, einer bewegbaren Haltevorrichtung 70 und einer Antriebseinrichtung 72. Ein Steuersignaleingang 74 der Antriebseinrichtung 72 ist mit dem Signalausgang 47 der Steuerung 40 gekoppelt. Die bewegbare Haltevorrichtung 70 ist bei dem in Figur 9 dargestellten Beispiel in der Art einer Vorrichtung, die gewöhnlich als Industrieroboter bezeichnet wird, dargestellt. Die bewegbare Haltevorrichtung 70 weist mehrere Gelenke mit jeweils einem oder mehreren rotatorischen Freiheitsgraden auf, denen mehrere Teile einer Antriebseinrichtung 72 zugeordnet sind. In Figur 9 ist die Antriebseinrichtung 72 vereinfachend nur an einem Gelenk 71 dargestellt.

Ein Bildschirm 60 ist durch die bewegbare Haltevorrichtung 70 gehalten. Gesteuert durch die Steuerung 40 und angetrieben durch die Antriebseinrichtung 72 kann die bewegbare Haltevorrichtung 70 Rotations- und Schwenkbewegungen 64, 66, 68 um drei Achsen und Translationsbewegungen 76, 78 in zwei orthogonale Richtungen entlang einer gekrümmten Fläche - angedeutet durch zwei Pfade 75, 77 - erzeugen.

Ähnlich wie anhand der Figuren 1 bis 5 dargestellt, empfängt die Steuerung 40 an ihrem Signaleingang 42 ein Signal, das die gegenwärtige Orientierung eines Endoskops (vgl. Figur 6) oder einer anderen Bilderfassungsvorrichtung repräsentiert oder aus dem die Steuerung 40 die gegenwärtige Orientierung des Endoskops oder der anderen Bilderfassungsvorrichtung bestimmen kann. Die Steuerung 40 steuert die Antriebseinrichtung 72 so, dass die Orientierung des Bildschirms 60 im Raum (vgl. Figur 7) jederzeit einer Soll-Orientierung, die der gegenwärtig vorliegenden Orientierung des Endoskops oder der anderen Bilderfassungsvorrichtung zugeordnet ist, entspricht. Optional steuert die Steuerung 40 die Antriebseinrichtung 72 so, dass auch die Position des Bildschirms (vgl. Figur 8) jederzeit einer Soll-Position, die der gegenwärtig vorliegenden Orientierung des Endoskops oder der anderen Bilderfassungsvorrichtung zugeordnet ist, entspricht.

Figur 10 zeigt eine schematische Darstellung von medizinischem Personal 12 an einer Konsole 90 eines Da-Vinci-Operationssystems oder eines ähnlichen Operationssystems. An der Konsole 90 sind mehrere Eingabegeräte 91, beispielsweise Joysticks, vorgesehen, mittels derer das medizinische Personal 12 das Operationssystem steuern kann. Teil des Operationssystems ist eine in den Körper eines Patienten einführbare und in Figur 10 nicht dargestellte Bilderfassungsvorrichtung, beispielsweise ein starres oder flexibles Endoskop. Ein von der Bilderfassungsvorrichtung erfasstes Bild wird durch einen Bildschirm 60 oder eine andere Bildwiedergabevorrichtung wiedergegeben. Die Orientierung der Bilderfassungsvorrichtung im Raum kann durch Rotieren oder Schwenken um eine oder mehrere Rotations- oder Schwenkachsen verändert werden. Die Orientierung der Bilderfassungsvorrichtung im Raum ist dem Operationssystem insbesondere aus der Steuerung derselben bekannt.

Der Bildschirm 60 wird von einer bewegbaren Haltevorrichtung 70 gehalten, die durch eine Antriebseinrichtung 72 bewegt werden kann. Ein Signaleingang 74 der Antriebseinrichtung 72 ist mit einem Signalausgang 47 einer Steuerung 40 gekoppelt. Die Steuerung 40, die bewegbare Haltevorrichtung 70 und die Antriebseinrichtung 72 bilden ein System mit ähnlichen Merkmalen, Eigenschaften und Funktionen wie die anhand der Figuren 1 bis 5 und 9 beschriebenen Systeme. Die Steuerung 40 stellt die Orientierung des Bildschirms 60 abhängig von der Orientierung der Bilderfassungsvorrichtung ein.

Figur 11 zeigt schematische Darstellungen eines Bildschirms 60 ähnlich dem Bildschirm der anhand der Figuren 1 bis 5, 9 und 10 dargestellten Systeme. In allen drei Darstellungen des Bildschirms 60 in Figur 11 ist an der Bildwiedergabefläche 62 ein Bild wiedergegeben, wie es bei der in Figur 6 dargestellten Situation von dem Endoskop 20 erfasst werden kann. Sichtbar sind jeweils das distale Ende des medizinischen Instruments 14 und das Organ 18 mit einer pfeilförmigen Struktur.

In Figur 11 links ist durch einen Pfeil 84 eine Rotation des auf dem Bildschirm 60 wiedergegebenen Bilds relativ zu der Bildwiedergabefläche 62 um die Flächennormale 63 der Bildwiedergabefläche 62 als Rotationsachse dargestellt. Diese Rotation 84 des wiedergegebenen Bilds relativ zu der Bildwiedergabefläche 62 kann eine Rotation des Bildschirms 60 um die Flächennormale 63 der Bildwiedergabefläche 62 des Bildschirms 60 (vergl. die Figuren 7 bis 10) ergänzen oder ersetzen.

In Figur 11 in der Mitte ist eine Verformung des an der Bildwiedergabefläche 62 wiedergegebenen Bilds angedeutet, die bei einem Betrachter den Eindruck einer Schwenkbewegung 86 des wiedergegebenen Bilds um eine horizontale Schwenkachse 85 erzeugt. Diese in Figur 11 in der Mitte dargestellte Verformung des wiedergegebenen Bilds kann eine Schwenkbewegung 66 des Bildschirms 60 um eine horizontale Schwenkachse 65 (vgl. Figuren 7 bis 10) ergänzen oder ersetzen.

In Figur 11 rechts ist eine Verformung des an der Bildwiedergabefläche 62 wiedergegebenen Bilds angedeutet, die bei einem Betrachter den Eindruck einer Schwenkbewegung 88 des wiedergegebenen Bilds um eine vertikale Schwenkachse 87 erzeugt. Die in Figur 11 rechts dargestellte Verformung des wiedergegebenen Bilds kann eine Schwenkbewegung 68 des Bildschirms 60 um eine vertikale Schwenkachse 67 (vgl. Figuren 7 bis 10) ergänzen oder ersetzen.

Indem eine Rotations- oder Schwenkbewegung 54, 56, 58 (vgl. Figur 6) einer Bilderfassungsvorrichtung 20 nur teilweise eine entsprechende Rotations- oder Schwenkbewegung 64, 66, 68 des Bildschirms 60, jedoch in Ergänzung dazu eine Rotation 84 und/oder eine Verformung des wiedergegebenen Bilds, die den Eindruck einer Schwenkbewegung 86, 88 erzeugt, bewirkt, kann ein ungünstiger Blickwinkel des medizinischen Personals 12 auf den Bildschirm 60 vermieden und/ oder eine einfachere, kostengünstigere, kompaktere und/oder einen geringeren Bauraum einnehmende Haltevorrichtung für den Bildschirm 60 ermöglicht werden.

Die in Figur 11 gezeigte Rotation und/oder Verformung des wiedergegebenen Bilds kann durch eine Bildverarbeitungseinrichtung erfolgen, die durch die Steuerung 40 (vgl. Figuren 1 bis 5, 9 und 10) gesteuert und/oder in diese integriert sein kann.

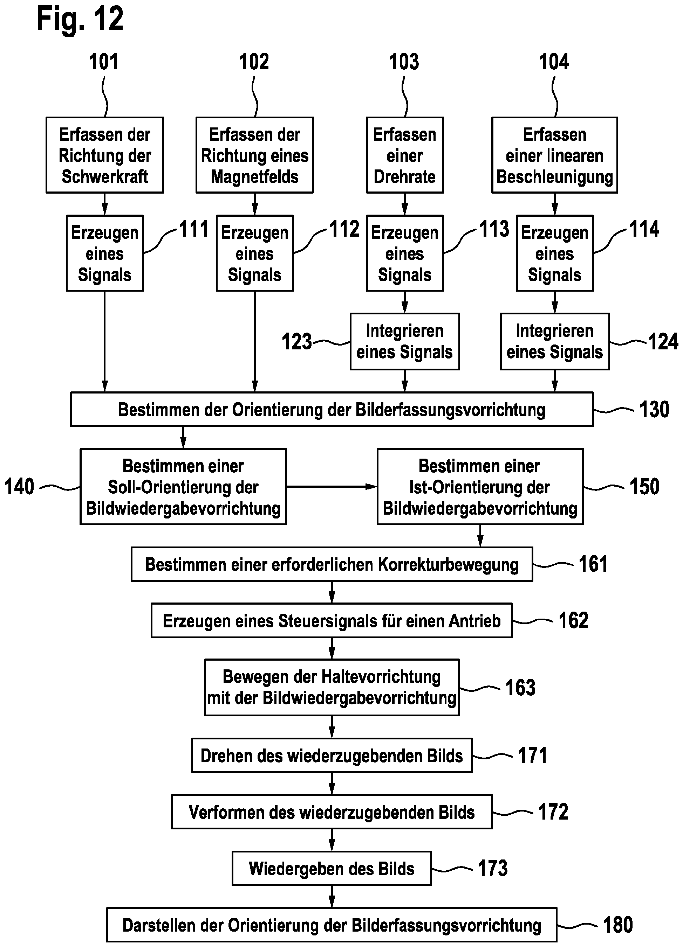

Figur 12 zeigt ein schematisches Flussdiagramm eines Verfahrens zum Wiedergeben eines mittels einer bewegbaren Bilderfassungsvorrichtung erfassten Bilds. Das Verfahren ist insbesondere mittels eines der anhand der Figuren 1 bis 11 dargestellten Systeme ausführbar. Deshalb werden nachfolgend beispielhaft Bezugszeichen aus den Figuren 1 bis 11 verwendet. Das Verfahren ist jedoch auch mit Systemen ausführbar, die sich in einem oder mehreren Merkmalen, Eigenschaften und Funktionen von den anhand der Figuren 1 bis 11 dargestellten Systemen unterscheiden.

Bei einem Schritt 101 wird durch einen Sensor 38, der in oder an einer Bilderfassungsvorrichtung 20 angeordnet und starr mit der Bilderfassungsvorrichtung 20 verbunden ist, eine Richtung der von der Erde erzeugten Gravitation erfasst. Gleichzeitig oder unmittelbar danach wird bei einem weiteren Schritt 111 ein Signal, das die Richtung der Schwerkraft relativ zu dem Sensor 38 repräsentiert, erzeugt. Aus dem erzeugten Signal kann deshalb auf die Orientierung der Bilderfassungsvorrichtung 20 relativ zur Vertikalen geschlossen werden.

Bei einem weiteren Schritt 102 wird durch einen Sensor 38, der in oder an einer Bilderfassungsvorrichtung 20 angeordnet und starr mit der Bilderfassungsvorrichtung 20 verbunden ist, die Richtung eines Magnetfelds relativ zu dem Sensor und damit relativ zu der Bilderfassungsvorrichtung 20 erfasst. Das Magnetfeld ist beispielsweise das Magnetfeld der Erde oder ein künstlich erzeugtes Magnetfeld. Bei einem gleichzeitig oder unmittelbar danach stattfindenden weiteren Schritt 112 wird ein Signal erzeugt, das die Richtung des Magnetfelds relativ zu dem Sensor 38 und damit relativ zu der Bilderfassungsvorrichtung 20 repräsentiert. Aus dem Signal kann auf die Orientierung der Sensors 38 und damit auf die Orientierung der Bilderfassungsvorrichtung 20 relativ zu dem Magnetfeld geschlossen werden.

Bei einem weiteren Schritt 103 wird durch einen Sensor 38, der in oder an einer Bilderfassungsvorrichtung 20 angeordnet und starr mit der Bilderfassungsvorrichtung 20 verbunden ist, eine Drehrate des Sensors 38 und damit auch der Bilderfassungsvorrichtung 20 erfasst. Dies kann ein Erfassen der Drehrate und der Richtung der momentanen Rotationsachse oder ein Erfassen von Drehraten bezogen auf mehrere orthogonale Bezugsachsen umfassen. Bei einem gleichzeitig oder unmittelbar danach stattfindenden Schritt 113 wird ein Signal erzeugt, das die beim Schritt 103 erfasste Drehrate repräsentiert. Bei einem nachfolgenden Schritt 123 wird das bei dem Schritt 113 erzeugte Signal über die Zeit integriert. Wenn die Winkelposition des Sensors 38 und der Bilderfassungsvorrichtung 20 zu einem Anfangszeitpunkt bekannt ist, kann zu jedem späteren Zeitpunkt aus dem integrierten Signal auf die vorliegende Winkelposition bzw. Orientierung des Sensors 38 und der Bilderfassungsvorrichtung 20 geschlossen werden.

Bei einem weiteren Schritt 104 wird durch einen Sensor 38, der in oder an einer Bilderfassungsvorrichtung 20 angeordnet und starr mit der Bilderfassungsvorrichtung 20 verbunden ist, eine lineare oder translatorische Beschleunigung erfasst. Das Erfassen der Beschleunigung kann ein Erfassen des Betrags und der Richtung der Beschleunigung umfassen. Alternativ kann das Erfassen der Beschleunigung ein Erfassen von mehreren Komponenten (beispielsweise parallel zu den Achsen eines lokalen oder globalen kartesischen Koordinatensystems) umfassen. Bei einem gleichzeitigen oder unmittelbar nachfolgenden Schritt 114 wird ein Signal erzeugt, das die erfasste Beschleunigung repräsentiert. Bei einem nachfolgenden Schritt 124 wird das erzeugte Signal integriert. Wenn die Position des Sensors 38 und der Bilderfassungsvorrichtung 20 zu einem Anfangszeitpunkt bekannt ist, kann zu jedem späteren Zeitpunkt aus dem integrierten Signal die zu dem späteren Zeitpunkt vorliegende Position des Sensors 38 und der Bilderfassungsvorrichtung 20 berechnet bzw. bestimmt werden.

Die Schritte 101 und 111, die Schritte 102 und 112, die Schritte 103, 113 und 123 und die Schritte 104, 114, 124 stellen Alternativen dar, die in einem Verfahren alternativ oder gleichzeitig vorhanden sein können.

Bei einem weiteren Schritt 130 wird die Orientierung der Bilderfassungsvorrichtung 20 aufgrund des oder der von dem Sensor 38 oder den Sensoren in oder an der Bilderfassungsvorrichtung 20 erzeugten Signals oder Signale bestimmt.

Bei einem weiteren Schritt 140 wird aus der Orientierung der Bilderfassungsvorrichtung 20 eine Soll-Orientierung einer Bildwiedergabevorrichtung 60 bestimmt. Die Soll-Orientierung der Bildwiedergabevorrichtung kann mit der Orientierung der Bilderfassungsvorrichtung identisch oder eine beliebige Funktion der Orientierung der Bilderfassungsvorrichtung sein.

Bei einem weiteren Schritt 150 wird eine Ist-Orientierung der Bildwiedergabevorrichtung 60 bestimmt, die beispielsweise aus der zurückgelegten Zahl von Schritten von Schrittmotoren oder von Positions- oder Winkelsensoren an Gelenken einer Haltevorrichtung, die die Bildwiedergabevorrichtung hält, bekannt sein kann.

Bei einem weiteren Schritt 161 wird aus der Soll-Orientierung und der Ist-Orientierung der Bildwiedergabevorrichtung 60 eine erforderliche Korrekturbewegung der Bildwiedergabevorrichtung 60 bestimmt.

Bei einem weiteren Schritt 162 wird aus der erforderlichen Korrekturbewegung ein Steuersignal für eine Antriebseinrichtung 72 der bewegbaren Haltevorrichtung 70 für die Bildwiedergabevorrichtung 60 erzeugt.

Der Schritt 130, der Schritt 140, der Schritt 150, der Schritt 161 und der Schritt 162 werden insbesondere durch eine Steuerung 40 ausgeführt, die Teil der Bilderfassungsvorrichtung 20 oder Teil der Antriebseinrichtung 72 für die bewegbare Haltevorrichtung oder Teil der Bildwiedergabevorrichtung 60 oder eine eigene Baugruppe sein kann.

Bei einem weiteren Schritt 163 wird die die Bildwiedergabevorrichtung 60 haltende Haltevorrichtung 70 entsprechend dem Steuersignal bewegt.

Bei einem weiteren Schritt 171 wird das durch die Bildwiedergabevorrichtung 60 wiederzugebende Bild gedreht. Bei einem weiteren Schritt 172 wird das durch die Bildwiedergabevorrichtung 60 wiederzugebende Bild verformt, um bei einem Betrachter den Eindruck eines Schwenkens des wiedergegebenen Bilds um eine Achse 85, 87 relativ zu der Bildwiedergabevorrichtung 60 zu erzeugen.

Der Schritt 163, der Schritt 171 und der Schritt 172 stellen Alternativen dar, die gemeinsam oder alternativ Teil des in Figur 12 gezeigten Verfahrens sein können.

Bei einem Schritt 173 wird das Bild durch die Bildwiedergabevorrichtung 60 wiedergegeben.

Bei einem weiteren und optionalen Schritt 180 wird die Orientierung der Bilderfassungsvorrichtung 20 im Raum graphisch oder numerisch an der Bildwiedergabevorrichtung 60 dargestellt. Dies kann beispielsweise durch eine perspektivische Darstellung eines Pfeils, der eine vorbestimmte Bezugsrichtung anzeigt, erfolgen. Die Bezugsrichtung ist abhängig von der medizinischen Maßnahme und ihrem Ort, beispielsweise kranial, kaudal, ventral oder dorsal. Alternativ kann die Orientierung der Bilderfassungsvorrichtung 20 im Raum beispielsweise durch eine verkleinerte und perspektivische Darstellung des Patientenkörpers am Rand der Bildwiedergabefläche 62 der Bildwiedergabevorrichtung 60 erfolgen. Alternativ oder zusätzlich kann die Orientierung der Bilderfassungsvorrichtung 20 im Raum durch Angabe von Winkeln an analogen Skalen oder in Form von Zahlenwerten erfolgen.

Die Schritte des anhand der Figur 12 dargestellten Verfahrens werden insbesondere zyklisch wiederholt, wobei einige Schritte gleichzeitig oder in anderer Reihenfolge als dargestellt ausgeführt werden können.

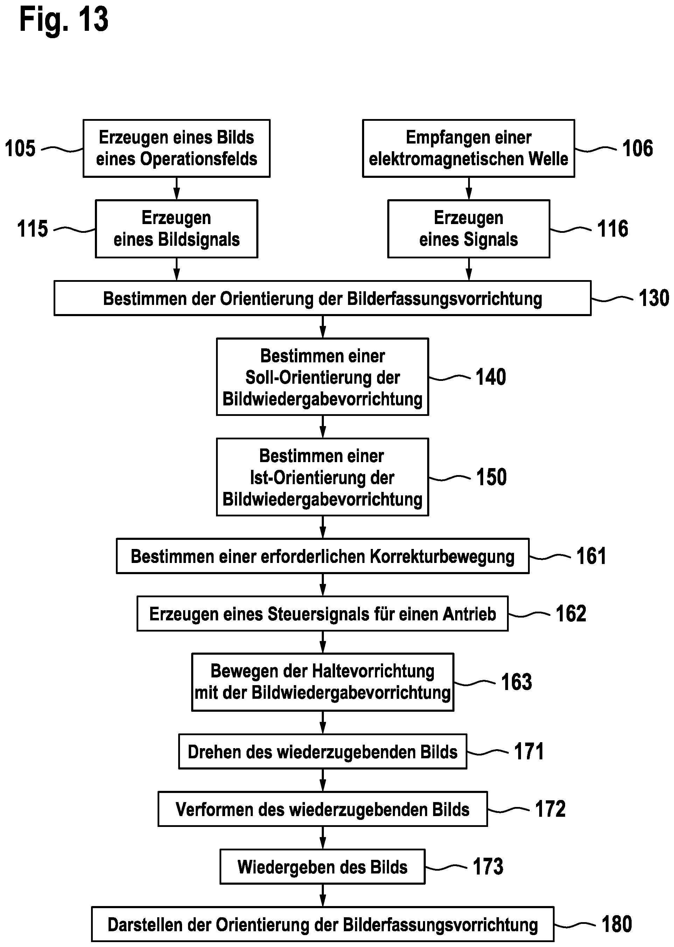

Figur 13 zeigt ein schematisches Flussdiagramm eines weiteren Verfahrens, das in mehreren Schritten, Eigenschaften und Funktionen dem anhand der Figur 12 dargestellten Verfahren ähnelt. Nachfolgend sind insbesondere Schritte beschrieben, in denen das in Figur 13 gezeigte Verfahren sich von dem anhand der Figur 12 dargestellten Verfahren unterscheidet.

Bei einem Schritt 105 wird durch eine Kamera 30 ein Bild eines Operationsfelds, in dem auch ein proximales Ende einer Bilderfassungsvorrichtung 20 angeordnet ist, erzeugt. Bei einem gleichzeitig oder unmittelbar danach erfolgenden Schritt 115 wird ein Bildsignal erzeugt, das das erfasste Bild repräsentiert.

Bei einem weiteren Schritt 106 wird eine elektromagnetische Welle, die von einem Transponder oder einem Sender 36 in oder an einer Bilderfassungsvorrichtung ausgeht, empfangen, insbesondere mittels einer Antenne oder einer anderen Empfangseinrichtung 34. Bei einem gleichzeitig oder unmittelbar danach ausgeführten weiteren Schritt 116 wird ein Signal erzeugt, das die Amplitude, Intensität oder Leistungsdichte und/oder die Polarisation und/oder eine Phase der empfangenen elektromagnetischen Welle repräsentiert. Aus dem Signal kann auf die Orientierung des Transponders oder Senders 36 und damit der Bilderfassungsvorrichtung 20 im Raum geschlossen werden.

Die Schritte 105, 115 und die Schritte 106, 116 stellen Alternativen dar. Ein Verfahren kann die Schritte 105, 115 und/oder die Schritte 106, 116 umfassen.

Bei einem weiteren Schritt 130 wird durch Auswertung des beim Schritt 115 erzeugten Bildsignals und/oder durch Auswerten des beim Schritt 116 erzeugten Signals die Orientierung der Bilderfassungsvorrichtung 20 im Raum bestimmt.

Die weiteren Schritte des in Figur 13 gezeigten Verfahrens entsprechen den bereits anhand der Figur 12 beschriebenen Schritten.

Die Schritte 101, 111 und/oder die Schritte 102, 112 und/oder die Schritte 103, 113, 123 und/ oder die Schritte 104, 114, 124 aus dem anhand der Figur 12 dargestellten Verfahren können mit den Schritten 105, 115 und/oder mit den Schritten 106, 116 aus dem anhand der Figur 13 dargestellten Verfahren kombiniert werden.

### Bezugszeichen

- 12: medizinisches Personal
- 14: medizinisches Instrument
- 16: Hohlraum im Körper eines Patienten
- 18: Organ oder anderes Objekt in dem Hohlraum 16 des Patienten
- 20: Endoskop als Bilderfassungsvorrichtung
- 22: distales Ende des Endoskops 20
- 24: proximales Ende des Endoskops 20
- 25: Kamera, mit dem proximalen Ende 24 des Endoskops 20 gekoppelt oder in das Endoskop 20 integriert
- 26: Struktur am proximalen Ende 24 zur Erkennung der Orientierung des Endoskops 20
- 28: Blickrichtung des Endoskops 20 und erste Rotationsachse
- 29: Rand des von dem Endoskop 20 erfassten Bereichs
- 30: Kamera zum optischen Erfassen des proximalen Endes 24 des Endoskops 20
- 32: Stativ für die Kamera 30
- 34: Einrichtung zur elektromagnetischen Erfassung der Orientierung des Endoskops 20
- 36: Transponder in oder an dem Endoskop 20
- 38: Sensor oder Transponder oder Sender an oder in dem Endoskop 20 (insbesondere Sensor für die Richtung der Schwerkraft oder des Erdmagnetfelds oder eines anderen magnetischen oder elektrischen Felds oder für eine Beschleunigung oder Drehratensensor)
- 40: Steuerung
- 42: Signaleingang der Steuerung 40, mit Kamera 30 oder Einrichtung 34 oder Sensor 38 gekoppelt
- 47: Signalausgang der Steuerung 40, mit Signaleingang 74 der Antriebseinrichtung 72 des motorisch bewegbaren Arms 70 gekoppelt
- 54: Rotationsbewegung des Endoskops 20 um dessen Blickrichtung 28 (erster rotatorischer Freiheitsgrad des Endoskops 20)
- 55: horizontale zweite Rotationsachse, orthogonal zu der Blickrichtung und ersten Rotationsachse 28 und zu der dritten Rotationsachse 57
- 56: Schwenk- oder Rotationsbewegung des Endoskops 20 um die zweite Rotationsachse 55 (zweiter rotatorischer Freiheitsgrad des Endoskops 20)
- 57: dritte Rotationsachse, orthogonal zu der Blickrichtung und ersten Rotationsachse 28 und zu der horizontalen zweiten Rotationsachse 55
- 58: Schwenk- oder Rotationsbewegung des Endoskops 20 um die dritte Rotationsachse 57 (dritter rotatorischer Freiheitsgrad des Endoskops 20)
- 60: Bildschirm als Bildwiedergabevorrichtung
- 62: Bildwiedergabefläche des Bildschirms 60
- 63: Flächennormale der Bildwiedergabefläche 62 und erste Rotationsachse des Bildschirms 60
- 64: Rotationsbewegung des Bildschirms 60 um die Flächennormale und erste Rotationsachse 63 (erster rotatorischer Freiheitsgrad des Bildschirms 60)
- 65: horizontale zweite Rotationsachse des Bildschirms 60, orthogonal zu der ersten Rotationsachse 63
- 66: Schwenk- oder Rotationsbewegung des Endoskops 20 um die zweite Rotationsachse 65 (zweiter rotatorischer Freiheitsgrad des Bildschirms 60)
- 67: dritte Rotationsachse des Bildschirms 60, orthogonal zu der ersten Rotationsachse 63 und zu der zweiten Rotationsachse 65
- 68: Schwenk- oder Rotationsbewegung des Endoskops 20 um die dritte Rotationsachse 67 (dritter rotatorischer Freiheitsgrad des Bildschirms 60)
- 70: bewegbarer Arm als bewegbare Haltevorrichtung mit mehreren Freiheitsgraden
- 71: Gelenk des bewegbaren Arms 70
- 72: Antriebseinrichtung für den bewegbaren Arm 70
- 74: Steuersignaleingang der Antriebseinrichtung 72
- 75: Pfad in einer vertikalen Ebene, entlang dessen der Bildschirm 60 bewegbar ist
- 76: translatorische Bewegung des Bildschirms 60 entlang des ersten Pfads 75 (erster translatorischer Freiheitsgrad des Bildschirms 60)
- 77: horizontaler Pfad, entlang dessen der Bildschirm 60 bewegbar ist
- 78: translatorische Bewegung des Bildschirms 60 entlang des zweiten Pfads 75 (zweiter translatorischer Freiheitsgrad des Bildschirms 60)
- 83: erste virtuelle Rotationsachse des auf dem Bildschirm 60 wiedergegebenen Bilds
- 84: erster virtueller rotatorischer Freiheitsgrad des auf dem Bildschirm 60 wiedergegebenen Bilds (virtuelle Rotation um die erste virtuelle Rotationsachse 83)
- 85: zweite virtuelle Rotationsachse des auf dem Bildschirm 60 wiedergegebenen Bilds, orthogonal zu der ersten virtuellen Rotationsachse 83 und horizontal
- 86: zweiter virtueller rotatorischer Freiheitsgrad des auf dem Bildschirm 60 wiedergegebenen Bilds (virtuelle Rotation um die zweite virtuelle Rotationsachse 85)
- 87: dritte virtuelle Rotationsachse des auf dem Bildschirm 60 wiedergegebenen Bilds, orthogonal zu der ersten virtuellen Rotationsachse 83 und zu der zweiten virtuellen Rotationsachse 85
- 88: dritter virtueller rotatorischer Freiheitsgrad des auf dem Bildschirm 60 wiedergegebenen Bilds (virtuelle Rotation um die dritte virtuelle Rotationsachse 87)
- 90: Konsole eines Operationssystems
- 91: Eingabegerät an der Konsole 90
- 101: Erfassen der Richtung der Schwerkraft und Erzeugen eines Sensorsignals
- 102: Erfassen der Richtung eines elektrischen oder magnetischen Felds
- 103: Erfassen einer linearen Beschleunigung
- 104: Erfassen einer Drehrate
- 105: Erzeugen eines Bildsignals
- 106: Empfangen einer elektromagnetischen Welle
- 111: Erzeugen eines Sensorsignals
- 112: Erzeugen eines Sensorsignals
- 113: Erzeugen eines Sensorsignals
- 114: Erzeugen eines Sensorsignals
- 115: Erzeugen eines Bildsignals
- 116: Erzeugen eines Signals
- 123: Integrieren des Sensorsignals
- 124: Integrieren des Sensorsignals
- 130: Bestimmen der vorliegenden Orientierung der Bilderfassungsvorrichtung 20
- 140: Bestimmen einer Soll-Orientierung der Bildwiedergabevorrichtung 60
- 150: Bestimmen einer Ist-Orientierung der Bildwiedergabevorrichtung 60
- 161: Bestimmen einer erforderlichen Korrekturbewegung
- 162: Erzeugen eines Steuersignals für einen Antrieb (72)
- 163: Bewegen der Haltevorrichtung 70 mit der Bildwiedergabevorrichtung (60) zu der Soll-Orientierung
- 171: Drehen des durch die Bildwiedergabevorrichtung 60 wiederzugebenden Bilds
- 172: Verformen des durch die Bildwiedergabevorrichtung 60 wiederzugebenden Bilds
- 173: Wiedergeben des Bilds durch die Bildwiedergabevorrichtung 60
- 180: graphisches oder numerisches Darstellen der Orientierung der Bilderfassungsvorrichtung 20 im Raum an der Bildwiedergabefläche 62

## Patentansprüche

1. **System** zum Halten einer Bildwiedergabevorrichtung (60) zum Wiedergeben eines mittels einer Bilderfassungsvorrichtung (20) erfassten Bilds, mit:
einer **bewegbaren Haltevorrichtung** (70) zum veränderbaren Halten der Bildwiedergabevorrichtung (60);
einer **steuerbaren Antriebseinrichtung** (72) zum Bewegen der Haltevorrichtung (70), mit einem Steuersignaleingang (74) zum Empfangen eines Steuersignals;
einer **Steuerung** (40) mit einem Signaleingang (42) zum Empfangen eines Signals, das eine Orientierung oder eine Änderung der Orientierung der Blickrichtung (28) der Bilderfassungsvorrichtung (20) im Raum repräsentiert oder eine Bestimmung der Orientierung oder der Änderung der Orientierung der Blickrichtung (28) der Bilderfassungsvorrichtung (20) ermöglicht, und einem mit dem Steuersignaleingang (74) der steuerbaren Antriebseinrichtung (72) koppelbaren Steuersignalausgang (47) zum Bereitstellen eines Steuersignals zum Steuern der steuerbaren Antriebseinrichtung (72),
wobei die Steuerung (40) vorgesehen und ausgebildet ist, um die steuerbare Antriebseinrichtung (72) so zu steuern, dass innerhalb eines vorbestimmten Bereichs möglicher Orientierungen der Blickrichtung (28) der Bilderfassungsvorrichtung (20) im Raum die **Orientierung der Bildwiedergabevorrichtung** (60) im Raum eine vorbestimmte **Funktion der Orientierung der Blickrichtung (28) der Bilderfassungsvorrichtung** (20) im Raum ist.

2. System nach dem vorangehenden Anspruch, bei dem die Steuerung (40) ausgebildet ist, um die Antriebseinrichtung (72) so zu steuern, dass innerhalb eines vorbestimmten Winkelbereichs ein **Schwenken** (56; 58) der **Blickrichtung** (28) der Bilderfassungsvorrichtung (20) um eine erste Achse (55; 57) ein **Schwenken** (66; 68) der Bildwiedergabevorrichtung (60) um eine zweite Achse (65; 67) bewirkt.

3. System nach einem der vorangehenden Ansprüche, bei dem die erste Achse (55) vertikal ist.

4. System nach einem der vorangehenden Ansprüche, bei dem die Steuerung (40) ausgebildet ist, um die Antriebseinrichtung (72) so zu steuern, dass innerhalb eines vorbestimmten Winkelbereichs ein Verändern (56) eines Winkels zwischen der Blickrichtung (28) der Bilderfassungsvorrichtung (20) und der Vertikalen eine Veränderung (66) des Winkels zwischen der Flächennormalen (63) einer Bildwiedergabefläche (62) der Bildwiedergabevorrichtung (60) und der Vertikalen bewirkt.

5. System nach einem der vorangehenden Ansprüche, bei dem die Steuerung (40) ausgebildet ist, um die Antriebseinrichtung (72) so zu steuern, dass hinsichtlich eines Schwenkens (56; 58) der Blickrichtung (28) der Bilderfassungsvorrichtung (20) um die erste Achse (55; 57) und eines Schwenkens (66; 68) der Bildwiedergabevorrichtung (20) um die zweite Achse (65; 67) innerhalb des vorbestimmten Winkelbereichs **jeder Winkelposition** der Blickrichtung (28) der Bilderfassungsvorrichtung (20) **eine Winkelposition** der Bildwiedergabevorrichtung (60) **zugeordnet** ist.

6. System nach einem der Ansprüche 2 bis 5, bei dem die Steuerung (40) ferner ausgebildet ist, um die Antriebseinrichtung (72) so zu steuern, dass ein **Schwenken** (56; 58) **der Blickrichtung** (28) der Bilderfassungsvorrichtung (20) um die erste Achse (55; 57) ferner ein **Bewegen** (76; 78) **der Bildwiedergabevorrichtung** (60) entlang eines **Pfads** (75; 77) bewirkt.

7. System nach einem der vorangehenden Ansprüche, bei dem die Steuerung (40) ferner ausgebildet ist, um die Antriebseinrichtung (72) so zu steuern, dass ein Schwenken (56) der Blickrichtung (28) der Bilderfassungsvorrichtung (20) um die erste horizontale Achse (55) ferner ein Bewegen (76) der Bildwiedergabevorrichtung (60) entlang eines Pfads (75), der zumindest in einem kleinen Abschnitt oder in einem Punkt vertikal oder im Wesentlichen vertikal ist, bewirkt.

8. System nach einem der vorangehenden Ansprüche, bei dem
der Signaleingang (42) der Steuerung (40) ferner vorgesehen und ausgebildet ist zum Empfangen eines Signals, das eine Position oder eine Änderung der Position der Bilderfassungsvorrichtung (20) repräsentiert oder eine Bestimmung der Position oder der Änderung der Position der Bilderfassungsvorrichtung (20) ermöglicht,
die Steuerung (40) ferner vorgesehen und ausgebildet ist, um die steuerbare Antriebseinrichtung (72) so zu steuern, dass innerhalb eines vorbestimmen Bereichs möglicher Positionen der Bilderfassungsvorrichtung (20) die **Position der Bildwiedergabevorrichtung** (60) eine vorbestimmte **Funktion der Position der Bilderfassungsvorrichtung** (20) ist.

9. System nach einem der vorangehenden Ansprüche, bei dem der Signaleingang (42) der Steuerung (40) zum Empfangen eines **Signals** oder eines über die Zeit integrierten Signals von einem Sensor (30, 34, 38) ausgebildet ist.

10. System nach einem der vorangehenden Ansprüche, bei dem
der Signaleingang (42) der Steuerung (40) zum Empfangen eines **Bildsignals** von einer Kamera (30) zum Erfassen eines Operationsfelds ausgebildet ist,
die Steuerung (40) zum Bestimmen der Orientierung der Bilderfassungsvorrichtung (20) durch Auswertung eines am Signaleingang (42) empfangenen Bildsignals vorgesehen und ausgebildet ist.

11. System nach einem der vorangehenden Ansprüche, bei dem
der Signaleingang (42) der Steuerung (40) zum Empfangen eines **Signals** von einem Empfänger (34) für elektromagnetische Wellen, die von der Bilderfassungsvorrichtung (20) ausgehen, ausgebildet ist,
die Steuerung (40) zum Bestimmen der Orientierung der Bilderfassungsvorrichtung (20) im Raum durch Auswertung des von dem Empfänger (34) erhaltenen Signals vorgesehen und ausgebildet ist.

12. System nach einem der vorangehenden Ansprüche, bei dem
die Steuerung (40) ausgebildet ist, um die Antriebseinrichtung (72) so zu steuern, dass innerhalb eines vorbestimmten Winkelbereichs eine **Rotation der Bilderfassungsvorrichtung** (20) **um deren Blickrichtung** (28) um einen ersten Winkel eine Rotation der Bildwiedergabevorrichtung (60) um einen zweiten Winkel bewirkt,
der zweite Winkel dem ersten Winkel gleicht oder ein vorbestimmter Bruchteil des ersten Winkels oder eine vorbestimmte Funktion des ersten Winkels oder eine vorbestimmte Funktion der Winkelposition der Bilderfassungsvorrichtung (20) ist.

13. **Verfahren** zum Wiedergeben eines mittels einer bewegbaren Bilderfassungsvorrichtung (20) erfassten Bilds, mit folgenden Schritten:
**Erfassen** (101, 102, 103, 104, 105, 106, 111, 112, 113, 114, 115, 116, 123, 124, 130) **der Orientierung** der Blickrichtung der Bilderfassungsvorrichtung (20) im Raum mittels eines Sensors (30; 34; 38);
**Einstellen** (161, 162, 163) **der Orientierung** der Bildwiedergabevorrichtung (60) im Raum abhängig von der Orientierung der Bilderfassungsvorrichtung (20) im Raum.

14. Verfahren nach dem vorangehenden Anspruch, ferner mit folgendem Schritt:
graphisches oder numerisches Darstellen (170) der Orientierung der Bilderfassungsvorrichtung (20) im Raum an einer Bildwiedergabefläche (62) der Bildwiedergabevorrichtung (60).

15. Verfahren nach einem der Ansprüche 13 und 14, ferner mit folgendem Schritt:
Verformen (182) des auf der Bildwiedergabefläche (62) dargestellten Bilds abhängig von einer Orientierung der Blickrichtung (28) der Bilderfassungsvorrichtung (20) im Raum.

## Claims

1. System for holding an image display apparatus (60) for displaying an image captured by means of an image capturing apparatus (20), comprising:
a movable holding apparatus (70) for an alterable hold of the image display apparatus (60);
a controllable drive device (72) for moving the holding apparatus (70), comprising a control signal input (74) for receiving a control signal;
a controller (40) comprising a signal input (42) for receiving a signal that represents an orientation or a change in the orientation of the viewing direction (28) of the image capturing apparatus (20) in space or that facilitates a determination of the orientation or the change in the orientation of the viewing direction (28) of the image capturing apparatus (20), and comprising a control signal output (47), couplable to the control signal input (74) of the controllable drive device (72), for providing a control signal for controlling the controllable drive device (72),
wherein the controller (40) is provided and configured to control the controllable drive device (72) in such a way that, within a predetermined range of possible orientations of the viewing direction (28) of the image capturing apparatus (20) in space, the orientation of the image display apparatus (60) in space is a predetermined function of the orientation of the viewing direction (28) of the image capturing apparatus (20) in space.

2. System according to the preceding claim, wherein the controller (40) is configured to control the drive device (72) in such a way that, within a predetermined angle range, pivoting (56; 58) of the viewing direction (28) of the image capturing apparatus (20) about a first axis (55; 57) causes pivoting (66; 68) of the image display apparatus (60) about a second axis (65; 67).

3. System according to one of the preceding claims, wherein the first axis (55) is vertical.

4. System according to one of the preceding claims, wherein the controller (40) is configured to control the drive device (72) in such a way that, within a predetermined angle range, altering (56) an angle between the viewing direction (28) of the image capturing apparatus (20) and the vertical causes altering (66) the angle between the surface normal (63) of an image display surface (62) of the image display apparatus (60) and the vertical.

5. System according to one of the preceding claims, wherein the controller (40) is configured to control the drive device (72) in such a way that, in respect of pivoting (56; 58) the viewing direction (28) of the image capturing apparatus (20) about the first axis (55; 57) and pivoting (66; 68) the image display apparatus (20) about the second axis (65; 67) within the predetermined angle range, every angle position of the viewing direction (28) of the image capturing apparatus (20) is associated with an angle position of the image display apparatus (60).

6. System according to one of the claims 2 through 5, wherein the controller (40) is further configured to control the drive device (72) in such a way that pivoting (56; 58) of the viewing direction (28) of the image capturing apparatus (20) about the first axis (55; 57) further causes a movement (76; 78) of the image display apparatus (60) along a path (75; 77).

7. System according to one of the preceding claims, wherein the controller (40) is further configured to control the drive device (72) in such a way that pivoting (56) of the viewing direction (28) of the image capturing apparatus (20) about the first horizontal axis (55) further causes a movement (76) of the image display apparatus (60) along a path (75) which is vertical or essentially vertical in a small sector or in one point.

8. System according to one of the preceding claims, wherein
the signal input (42) of the controller (40) is further provided and configured to receive a signal that represents a position or a change in the position of the image capturing apparatus (20) or that facilitates a determination of the position or the change in the position of the image capturing apparatus (20),
the controller (40) is further provided and configured to control the controllable drive device (72) in such a way that, within a predetermined range of possible positions of the image capturing apparatus (20), the position of the image display apparatus (60) is a predetermined function of the position of the image capturing apparatus (20).

9. System according to one of the preceding claims, wherein the signal input (42) of the controller (40) is configured to receive from a sensor (30, 34, 38) a signal or a signal that has been integrated over time.

10. System according to one of the preceding claims, wherein
the signal input (42) of the controller (40) is configured to receive an image signal from a camera (30) for capturing a surgical field,
the controller (40) is provided and configured to determine the orientation of the image capturing apparatus (20) by evaluating an image signal received at the signal input (42).

11. System according to one of the preceding claims, wherein
the signal input (42) of the controller (40) is configured to receive a signal from a receiver (34) for electromagnetic waves which emanate from the image capturing apparatus (20),
the controller (40) is provided and configured to determine the orientation of the image capturing apparatus (20) in space by evaluating the signal received from the receiver (34).

12. System according to one of the preceding claims, wherein
the controller (40) is configured to control the drive device (72) in such a way that, within a predetermined angle range, a rotation of the image capturing apparatus (20) through a first angle about its viewing direction (28) causes a rotation of the image display apparatus (60) through a second angle,
the second angle equals the first angle or is a predetermined fraction of the first angle or a predetermined function of the first angle or a predetermined function of the angle position of the image capturing apparatus (20).

13. Method of displaying an image captured by means of a movable image capturing apparatus (20), including the following steps:
capturing (101, 102, 103, 104, 105, 106, 111, 112, 113, 114, 115, 116, 123, 124, 130) the orientation of the viewing direction of the image capturing apparatus (20) in space by means of a sensor (30; 34; 38);
setting (161, 162, 163) the orientation of the image display apparatus (60) in space depending on the orientation of the image capturing apparatus (20) in space.

14. Method according to the preceding claim, further including the following step:
presenting (170) the orientation of the image capturing apparatus (20) in space graphically or numerically on an image display surface (62) of the image display apparatus (60).

15. Method according to one of the claims 13 and 14, further including the following step:
deforming (182) the image presented on the image display surface (62) depending on an orientation of the viewing direction (28) of the image capturing apparatus (20) in space.

## Revendications

1. Système de retenue d'un dispositif de reproduction d'images (60) permettant de reproduire une image acquise au moyen d'un dispositif d'acquisition d'image (20), comprenant :
un dispositif de retenue mobile (70) pour retenir de manière variable le dispositif de reproduction d'images (60) ;
un dispositif d'entraînement pouvant être commandé (72) pour déplacer le dispositif de retenue (70), comprenant une entrée de signal de commande (74) pour recevoir un signal de commande ;
un contrôleur (40) ayant une entrée de signal (42) pour recevoir un signal qui représente une orientation ou une modification de l'orientation de la direction de visée (28) du dispositif d'acquisition d'image (20) dans l'espace ou qui permet une détermination de l'orientation ou de la modification de l'orientation de la direction de visée (28) du dispositif d'acquisition d'image (20), et ayant une sortie de signal de commande (47) pouvant être couplée à l'entrée de signal de commande (74) du dispositif d'entraînement pouvant être commandé (72) pour fournir un signal de commande pour commander le dispositif d'entraînement pouvant être commandé (72),
dans lequel le contrôleur (40) est prévu et réalisé pour commander le dispositif d'entraînement pouvant être commandé (72) de telle sorte qu'à l'intérieur d'une plage prédéterminée d'orientations possibles de la direction visée (28) du dispositif d'acquisition d'image (20) dans l'espace, l'orientation du dispositif de reproduction d'images (60) dans l'espace est une fonction prédéterminée de l'orientation de la direction de visée (28) du dispositif d'acquisition d'image (20) dans l'espace.

2. Système selon la revendication précédente, dans lequel le contrôleur (40) est réalisé pour commander le dispositif d'entraînement (72) de telle sorte qu'à l'intérieur d'une plage angulaire prédéterminée, un pivotement (56 ; 58) de la direction de visée (28) du dispositif d'acquisition d'image (20) autour d'un premier axe (55 ; 57) provoque un pivotement (66 ; 68) du dispositif de reproduction d'images (60) autour d'un deuxième axe (65 ; 67).

3. Système selon l'une quelconque des revendications précédentes, dans lequel le premier axe (55) est vertical.

4. Système selon l'une quelconque des revendications précédentes, dans lequel le contrôleur (40) est réalisé pour commander le dispositif d'entraînement (72) de telle sorte qu'à l'intérieur d'une plage angulaire prédéterminée, une modification (56) d'un angle entre la direction de visée (28) du dispositif d'acquisition d'image (20) et la verticale provoque une modification (66) de l'angle entre la normale à la surface (63) d'une surface de reproduction d'images (62) du dispositif de reproduction d'images (60) et la verticale.

5. Système selon l'une quelconque des revendications précédentes, dans lequel le contrôleur (40) est réalisé pour commander le dispositif d'entraînement (72) de telle sorte que concernant un pivotement (56 ; 58) de la direction de visée (28) du dispositif d'acquisition d'image (20) autour du premier axe (55 ; 57) et un pivotement (66 ; 68) du dispositif de reproduction d'images (20) autour du deuxième axe (65 ; 67) à l'intérieur de la plage angulaire prédéterminée, une position angulaire du dispositif de reproduction d'images (60) est associée à chaque position angulaire de la direction de visée (28) du dispositif d'acquisition d'image (20).

6. Système selon l'une quelconque des revendications 2 à 5, dans lequel le contrôleur (40) est en outre réalisé pour commander le dispositif d'entraînement (72) de telle sorte qu'un pivotement (56 ; 58) de la direction de visée (28) du dispositif d'acquisition d'image (20) autour du premier axe (55 ; 57) provoque en outre un déplacement (76 ; 78) du dispositif de reproduction d'images (60) le long d'un chemin (75 ; 77).

7. Système selon l'une quelconque des revendications précédentes, dans lequel le contrôleur (40) est en outre réalisé pour commander le dispositif d'entraînement (72) de telle sorte qu'un pivotement (56) de la direction de visée (28) du dispositif d'acquisition d'image (20) autour du premier axe horizontal (55) provoque en outre un déplacement (76) du dispositif de reproduction d'images (60) le long d'un chemin (75) qui est vertical ou substantiellement vertical au moins sur une petite partie ou en un point.

8. Système selon l'une quelconque des revendications précédentes, dans lequel
l'entrée de signal (42) du contrôleur (40) est en outre prévue et réalisée pour recevoir un signal qui représente une position ou une modification de la position du dispositif d'acquisition d'image (20) ou qui permet de déterminer la position ou la modification de la position du dispositif d'acquisition d'image (20),
le contrôleur (40) est en outre prévu et réalisé pour commander le dispositif d'entraînement pouvant être commandé (72) de telle sorte qu'à l'intérieur d'une plage prédéterminée de positions possibles du dispositif d'acquisition d'image (20), la position du dispositif de reproduction d'images (60) est une fonction prédéterminée de la position du dispositif d'acquisition d'image (20).

9. Système selon l'une quelconque des revendications précédentes, dans lequel l'entrée de signal (42) du contrôleur (40) est réalisée pour recevoir d'un capteur (30, 34, 38) un signal ou un signal intégré par rapport au temps.

10. Système selon l'une quelconque des revendications précédentes, dans lequel
l'entrée de signal (42) du contrôleur (40) est réalisée pour recevoir un signal d'image d'une caméra (30) pour acquérir un champ d'opération,
le contrôleur (40) est prévu et réalisé pour déterminer l'orientation du dispositif d'acquisition d'image (20) par l'évaluation d'un signal d'image reçu à l'entrée de signal (42).

11. Système selon l'une quelconque des revendications précédentes, dans lequel
l'entrée de signal (42) du contrôleur (40) est réalisée pour recevoir un signal d'un récepteur (34) pour des ondes électromagnétiques qui émanent du dispositif d'acquisition d'image (20),
le contrôleur (40) est prévu et réalisé pour déterminer l'orientation du dispositif d'acquisition d'image (20) dans l'espace par l'évaluation du signal reçu par le récepteur (34).

12. Système selon l'une quelconque des revendications précédentes, dans lequel
le contrôleur (40) est réalisé pour commander le dispositif d'entraînement (72) de telle sorte qu'à l'intérieur d'une plage angulaire prédéterminée, une rotation du dispositif d'acquisition d'image (20) autour de sa direction de visée (28) d'un premier angle provoque une rotation du dispositif de reproduction d'images (60) d'un deuxième angle,
le deuxième angle est égal au premier angle ou est une fraction prédéterminée du premier angle ou une fonction prédéterminée du premier angle ou une fonction prédéterminée de la position angulaire du dispositif d'acquisition d'image (20).

13. Procédé permettant de reproduire une image acquise au moyen d'un dispositif d'acquisition d'image mobile (20), comprenant les étapes consistant à :
détecter (101, 102, 103, 104, 105, 106, 111, 112, 113, 114, 115, 116, 123, 124, 130) l'orientation de la direction de visée du dispositif d'acquisition d'image (20) dans l'espace au moyen d'un capteur (30 ; 34 ; 38) ;
régler (161, 162, 163) l'orientation du dispositif de reproduction d'images (60) dans l'espace en fonction de l'orientation du dispositif d'acquisition d'image (20) dans l'espace.

14. Procédé selon la revendication précédente, comprenant en outre l'étape suivante consistant à :
représenter de manière graphique ou numérique (170) l'orientation du dispositif d'acquisition d'image (20) dans l'espace sur une surface de reproduction d'images (62) du dispositif de reproduction d'images (60).

15. Procédé selon l'une quelconque des revendications 13 et 14, comprenant en outre l'étape suivante consistant à :
déformer (182) l'image représentée sur la surface de reproduction d'images (62) en fonction d'une orientation de la direction de visée (28) du dispositif d'acquisition d'image (20) dans l'espace.
